# EUROPÄISCHE PATENTANMELDUNG

(11) **EP 2 241 305 A2**
(43) Veröffentlichungstag der Anmeldung: **20.10.2010**
(21) Anmeldenummer: 09178546.9
(22) Anmeldetag: 09.12.2009
(51) Int. Cl.: A61K 8/55, A61Q 19/08

(54) **Antioxidative Zusammensetzungen**

(30) Priorität: 11.12.2008 DE 102008061340
(71) Anmelder: Henkel AG & Co. KGaA, 40589 Düsseldorf (DE)
(72) Erfinder: Jansen, Frank, 41470, Neuss (DE)

(57) **Zusammenfassung**

Die Erfindung betrifft die Verwendung einer antioxidativen Substanz, die einen antioxidativen Rest trägt, welcher über eine Linker-Gruppe mit einer lipophilen, kationisch geladenen Gruppe verknüpft ist, zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung sowie Zusammensetzungen, enthaltend eine solche antioxidative Substanz und 0,0001 bis 99 Gew.-% mindestens eines Tensids.

## Beschreibung

Die Erfindung betrifft die Verwendung einer antioxidativen Substanz, die einen antioxidativen Rest trägt, welcher über eine Linker-Gruppe mit einer lipophilen, kationisch geladenen Gruppe verknüpft ist, zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung sowie Zusammensetzungen, enthaltend eine solche antioxidative Substanz und 0,0001 bis 99 Gew.-% mindestens eines Tensids.

Mittel und Behandlungsmethoden zur Behandlung reifer oder intrinsisch oder extrinsisch gealterter oder lichtgeschädigter Haut sind eine bedeutende kosmetische Herausforderung. Bei diesen Mitteln besteht ein Bedarf nach Inhaltsstoffen, die den Zubereitungen weiteren Nutzen verleihen, sei es aus anwendungstechnischer Sicht, aus herstelltechnischer Sicht oder aus Sicht des Verbrauchers, der beispielsweise aufgrund bestimmter Inhaltsstoffe ein verbessertes subjektives Empfinden dem kosmetischen Mittel gegenüber entwickelt. Der Schwerpunkt der Leistung eines Inhaltsstoffes kann dabei je nach kosmetischem Mittel auch unterschiedlich sein. So kann ein und derselbe Stoff in einem Shampoo oder einer Spülung pflegende Eigenschaften für die behandelten Haare bewirken, während er in einem Haarfärbemittel darüber hinaus die Anfärbung der Kopfhaut verringert oder die zum Teil aggressive Wirkung des Färbemittels kompensiert.

Die Altershaut wird kosmetisch in erster Linie mit Vitamin A-Derivaten oder Hydroxysäuren behandelt, die über eine Stimulation der Proliferation der Basalzellen in der Epidermis zu einer Verdickung der Epidermis und damit Glättung der Haut führen.

Retinoide greifen in den Metabolismus der Hautzellen ein und bewirken neben der Anregung der Proliferation und Differenzierung der epidermalen Keratinozyten die Steigerung der Collagenproduktion durch Fibroblasten. Darüber hinaus soll Retinol die Bildung von Collagen verdauenden Enzymen reduzieren (New Scientist 2031, 42-46, 1996). Retinsäure besitzt jedoch teratogene Eigenschaften und darf nur in verschreibungspflichtigen Pharmaka eingesetzt werden. Der Einsatz von Retinol in kosmetischen und pharmazeutischen topischen Praeparaten ist aus mehreren Gründen als problematisch zu betrachten. So weist Retinol eine relative hohe Phototoxizität auf und kann deshalb nur in geringen Konzentrationen in Zusammensetzungen zur Anwendung am Menschen eingesetzt werden. Darüber hinaus wird Retinol unter Einwirkung von Wärme und/oder Licht leicht oxidativ abgebaut und ist in kosmetischen und pharmazeutischen Formulierungen schwierig zu stabilisieren.

Hydroxysäuren sind in den für die Behandlung der Hautalterung notwendigen Konzentrationen häufig schlecht verträglich, insbesondere für die Behandlung empfindlicher Haut und für die Behandlung der Haut im Augenbereich.

Neuere Ansätze bestehen darin, die Proteine, die in der trockenen oder der Altershaut fehlen oder vermindert vorkommen, gezielt zu substituieren bzw. indirekt in die Stoffwechselprozesse einzugreifen, die bei trockener Haut oder mit zunehmendem Alter gestört sind, um diese zu normalisieren. Als Beispiel sei hier die Stimulation der Collagensynthese mit dem Zweck der Faltenreduzierung angeführt. Weiterhin werden beispielsweise Laminin, Substanzen zur Verlängerung der Lebenszeit von Hautzellen und bestimmte Extrakte zur Stimulierung der epidermalen Differenzierung eingesetzt. Hierbei handelt es sich teilweise jedoch um pharmakologisch wirksame Substanzen mit hohem Nebenwirkungspotenzial.

Um eine besonders starke Hautalterung zu bekämpfen und zu verhindern, müssen die Zellen der Haut vor den Einflüssen von UV-Strahlung geschützt werden. Weiterhin müssen die Zellen in die Lage versetzt werden, solche Schäden, die durch UV-Strahlung hervorgerufen werden und somit zu einer stärkeren extrinsischen Hautalterung führen, selbst zu reparieren bzw. zu vermindern und so die Hautalterung einzudämmen.

In der US 6331532, der US7232809 und der US20080161267 werden antioxidative Substanzen beschrieben, die zur Behandlung von degenerativen Krankheiten, wie u.a. Parkinson oder Alzheimer, eingesetzt werden sollen. Eine topische Applikation auf der Haut oder eine Verwendung der beschriebenen antioxidativen Substanzen zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung wurde nicht beschrieben.

Eine Aufgabe der vorliegenden Erfindung ist es, gut verträgliche Wirkstoffe und Zusammensetzungen, die diese gut verträglichen Wirkstoffe enthalten, zur Verfügung zu stellen, um die Hautalterung zu bekämpfen.

Eine weitere Aufgabe der vorliegenden Erfindung ist es, gut verträgliche Wirkstoffe und Zusammensetzungen, die diese gut verträglichen Wirkstoffe enthalten, zur Verfügung zu stellen, um die Haut vor Schäden durch ultraviolette Strahlung zu schützen.

Eine weitere Aufgabe der vorliegenden Erfindung ist es, gut verträgliche Wirkstoffe und Zusammensetzungen, die diese gut verträglichen Wirkstoffe enthalten, zur Verfügung zu stellen, um eine Verbesserung der Elastizität der Haut zu erreichen.

Eine weitere Aufgabe der vorliegenden Erfindung ist es, gut verträgliche Wirkstoffe und Zusammensetzungen, die diese gut verträglichen Wirkstoffe enthalten, zur Verfügung zu stellen, um eine Verringerung und/oder Verhinderung der Faltenbildung zu erreichen.

Überraschend wurde gefunden, dass die vorliegende Zusammensetzung die gestellten Aufgaben in hervorragender Weise löst.

Gegenstand der vorliegenden Erfindung ist eine Zusammensetzung, enthaltend
a) mindestens eine antioxidative Substanz, die
   i) einen antioxidativen Rest (A) trägt, welcher über eine Linker-Gruppe (L) mit einer lipophilen, kationisch geladenen Gruppe (K) verknüpft ist, und
   ii) ein kosmetisch und/oder pharmazeutisch akzeptables Gegenion (Z) umfasst, sowie

b) 0,0001 bis 99 Gew.-%, bezogen auf das Gesamtgewicht der Zusammensetzung, mindestens eines Tensids.

Die Struktur der antioxidative Substanz ist in der Formel (AOS-I) beschrieben:

A-L-K⁺ Z⁻ (AOS-I)

Der antioxidative Rest (A) ist dabei kovalent mit der Linker-Gruppe (L) verbunden, die wiederum kovalent mit der lipophilen, kationisch geladenen Gruppe (K) verbunden ist. Solchermaßen aufgebaute antioxidative Substanzen sind überraschender Weise in der Lage, durch die Zellwände der Mitochondrien in die Mitochondrien selbst zu gelangen und dort den Alterungsprozeß positiv zu beeinflussen, insbesondere zu verlangsamen. Überraschenderweise wurde nun gefunden, dass der Einsatz nicht nur systemisch sondern auch topisch erfolgen kann. Eine verbesserte Verfügbarkeit von Substanzen mit antioxidativer Wirkung in den Mitochondrien der Fibroblasten führt zu einer verlängerten Lebenszeit der Fibroblasten, die die extracelluläre Matrix herstellen. Die Reduktion der extrazellulären Matrix in der Dermis führt zu Faltenbildung, Verlust der Elastizität der Haut sowie zu allgemeiner Hautalterung. Durch eine verbesserte Versorgung der Mitochondrien (als Kraftwerke der Fibroblasten) mit antioxidativen Substanzen kann die volle Funktionsfähigkeit der einzelnen Zelle länger gewährleistet werden und die negativen Begleiterscheinungen des Älterwerdens können herausgezögert werden. Somit sind die erfindungsgemäßen Zusammensetzungen als Anti-Aging-Mittel für die Haut besonders gut geeignet.

Weiterhin wurde überraschenderweise gefunden, dass die topische Applikation einer Zusammensetzung, die eine erfindungsgemäß aufgebaute antioxidative Substanz sowie 0,0001 bis 99 Gew.-% mindestens eines Tensids enthält, zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung geeignet ist.

Die erfindungsgemäße Zusammensetzung enthält 0,0001 bis 99 Gew.-%, bezogen auf das Gesamtgewicht der Zusammensetzung, mindestens eines Tensids.

Unter dem Begriff Tensid werden oberflächenaktive Substanzen verstanden, die eine anionische oder kationische Ladung im Molekül tragen. Ebenfalls kann im Molekül sowohl eine anionische als auch kationische Ladung vorhanden sein. Diese zwitterionischen oder amphoteren oberflächenaktiven Substanzen können erfindungsgemäß ebenfalls eingesetzt werden. Weiterhin können die oberflächenaktiven Substanzen auch nicht-ionisch sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe, lineare und verzweigte Fettsäuren mit 8 bis 30 C-Atomen (Seifen), Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist, Acylsarcoside mit 8 bis 24 C-Atomen in der Acylgruppe, Acyltauride mit 8 bis 24 C-Atomen in der Acylgruppe, Acylisethionate mit 8 bis 24 C-Atomen in der Acylgruppe, Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, lineare Alkansulfonate mit 8 bis 24 C-Atomen, lineare Alpha-Olefinsulfonate mit 8 bis 24 C-Atomen, Alpha-Sulfofettsäuremethylester von Fettsäuren mit 8 bis 30 C-Atomen, Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist, Gemische oberflächenaktiver Hydroxysulfonate gemäß DE-A-37 25 030, sulfatierte Hydroxyalkylpolyethylen- und/oder Hydroxyalkylenpropylenglykolether gemäß DE-A-37 23 354, Sulfonate ungesättigter Fettsäuren mit 8 bis 24 C-Atomen und 1 bis 6 Doppelbindungen gemäß DE-A-39 26 344, Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
Alkyl- und/oder Alkenyletherphosphate der Formel (VI), 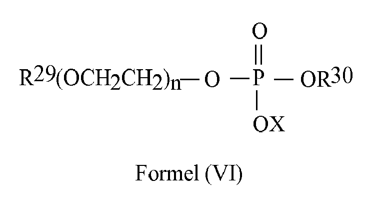 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
sulfatierte Fettsäurealkylenglykolester der Formel R³⁵CO(AlkO)ₙSO₃M, in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
Monoglyceridsulfate und Monoglyceridethersulfate der Formel (VIII), 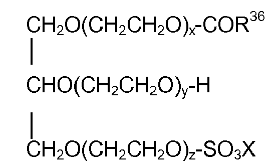 wie sie beispielsweise in EP 561825 B1, EP 561999 B1, DE 4204700 A1 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind, in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vor-zugsweise werden Monoglyceridsulfate der Formel (VIII) eingesetzt, in der R³⁶CO für einen linearen Acylrest mit 8 bis 18 Kohlenstoffatomen steht.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül und Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, z. B. das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie z. B. die unter den Verkaufsbezeichnungen Dehydol^{®} LS, Dehydol^{®} LT (Cognis) erhältlichen Typen, C₁₂-C₃₀-Fettsäuremono- und -diester von Anlagerungsprodukten von 1 bis 30 Mol Ethylenoxid an Glycerin, Anlagerungsprodukte von 5 bis 60 Mol Ethylenoxid an Rizinusöl und gehärtetes Rizinusöl, Polyolfettsäureester, wie beispielsweise das Handelsprodukt Hydagen^{®} HSP (Cognis) oder Sovermol-Typen (Cognis), alkoxylierte Triglyceride, alkoxylierte Fettsäurealkylester der Formel R³⁷CO-(OCH₂CHR³⁸)_{w}OR³⁹, in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht, Aminoxide, Hydroxymischether, wie sie beipielsweise in der DE-OS 19738866 beschrieben sind, Sorbitanfettsäureester und Anlagerungeprodukte von Ethylenoxid an Sorbitanfettsäureester wie beispielsweise die Polysorbate, Zuckerfettsäureester und Anlagerungsprodukte von Ethylenoxid an Zuckerfettsäureester, Anlagerungsprodukte von Ethylenoxid an Fettsäurealkanolamide und Fettamine, Fettsäure-N-alkylglucamide, Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind solche Alkylpolyglykoside, bei denen R
- im wesentlichen aus C₈- und C₁₀-Alkylgruppen oder
- im wesentlichen aus C₁₂- und C₁₄-Alkylgruppen oder
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 C-Atomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten. Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind z. B. 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette. Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält. Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein. Die Tenside werden in Mengen von 0,0001 - 99 Gew.%, bevorzugt 0,1 - 30 Gew.%, weiter bevorzugt 0,5-15 Gew.%, weiter bevorzugt 1,0 bis 10 Gew.-%, weiter bevorzugt 1,5-5 Gew.-%, jeweils bezogen auf das Gesamtgewicht der Zusammensetzung, eingesetzt. Werden zwei oder mehr Tenside eingesetzt beziehen sie die genannten Mengenbezeichnungen auf die Summe aller Tenside.

In einer bevorzugten Ausführungsform können nicht-ionische, zwitterionische und/oder amphotere Tenside sowie deren Mischungen bevorzugt sein.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäß verwendeten Mitteln bevorzugt in Mengen von 0,05 bis 10 Gew.-%, besonders bevorzugt von 0,1 bis 5 Gew.-%, jeweils bezogen auf das gesamte Mittel, enthalten.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10-15 können erfindungsgemäß besonders bevorzugt sein.

Unter den genannten Emulgatoren-Typen können die Emulgatoren, die kein Ethylenoxid und/oder Propylenoxid im Molekül enthalten, ganz besonders bevorzugt sein.

Gemäß einer besonders bevorzugten Ausführungsform der Erfindung ist mindestens ein Tensid, ausgewählt aus der Gruppe der nichtionischen Tenside, enthalten. Es können neben dem mindestens einen nichtionischen Tensid auch weitere Tenside in der erfindungsgemäßen Zusammensetzung enthalten sein, die aus anderen, insbesondere den oben genannten Tensidklassen ausgewählt sind.

Bevorzugte Zusammensetzungen, enthaltend mindestens ein nicht-ionisches Tensid, sind vorteilhafterweise besonders schonende und hautverträgliche kosmetische und/oder dermatologische Formulierungen, die eine geringe Hautreizung aufweisen, aber gleichzeitig die Penetration der antioxidativen Substanz in die Haut, insbesondere in die tieferen Hautschichten zu den Fibroblasten der Dermis, und dabei insbesondere in die Mitochondrien der Fibroblasten gewährleisten. Ihr Einsatz führt zu einer synergistischen Erhöhung der Wirksamkeit der erfindungsgemäßen antioxidativen Substanzen zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung

Gemäß einer besonders bevorzugten Ausführungsform der Erfindung ist das mindestens eine nichtionische Tensid ausgewählt aus
- Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- C_{12/18}-Fettsäuremono- und -diestern von Anlagerungsprodukten von 1 bis 30 Mol Ethylenoxid an Glycerin;
- Glycerinmono- und -diestern und Sorbitanmono- und -diestern von gesättigten und ungesättigten Fettsäuren mit 6 bis 22 Kohlenstoffatomen und deren Ethylenoxidanlagerungsprodukten;
- Alkylmono- und -oligoglycosiden mit 8 bis 22 Kohlenstoffatomen im Alkylrest und deren ethoxylierten Analoga;
- Anlagerungsprodukten von 15 bis 60 Mol Ethylenoxid an Ricinusöl und/oder gehärtetes Ricinusöl;
- Polyolestern;
- Anlagerungsprodukten von 2 bis 15 Mol Ethylenoxid an Ricinusöl und/oder gehärtetes Ricinusöl;
- Partialestern auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkoholen, Alkylglucosiden sowie Polyglucosiden;
- Trialkylphosphaten sowie Mono-, Di- und/oder Tri-PEG-Alkylphosphaten;
- Wollwachsalkoholen;
- Polysiloxan-Polyalkyl-Polyether-Copolymeren bzw. entsprechenden Derivaten;
- Mischestern aus Pentaerythrit, Fettsäuren, Citronensäure und Fettalkohol und/oder Mischestern von Fettsäuren mit 6 bis 22 Kohlenstoffatomen, Methylglucose und Polyolen sowie
- Polyalkylenglycolen.

Gemäß einer bevorzugten Ausführungsform der Erfindung ist die lipophile, kationisch geladene Gruppe (K) der antioxidativen Substanz ausgewählt aus substituierten oder unsubstituierten Triphenylphosphoniumkationen.

Substituierte Triphenylphosphoniumkationen sind an einem, zwei oder drei der drei Phenylreste an ein, zwei, drei Positionen. Bevorzugt werden die meta- bzw. para-Positionen der Phenylreste (an den Positionen 2 oder 4 (meta) bzw. 3 (para) substituiert.

Bevorzugt sind hydrophobe Reste wie beispielsweise substituierte oder unsubstituierte Alkyl-, Akenyl- und/oder Alkinylreste. Besonders bevorzugt sind unsubstituierte unverzweigte oder verzweigte Alkylreste. Insbesondere geeignet sind Methyl-, Ethyl-, Propyl-, iso-Propyl-, Butyl- oder tert.-Butylreste.

Bevorzugt sind hierbei die ein- und/oder zweifache Substitution an einem, zwei oder drei Phenylresten. Zu beachten ist dabei, dass keine sterische Hinderung auftritt.

Besonders bevorzugt ist sind antioxidative Reste (A) mit einem unsubstituierten Triphenylphosphoniumkation (im weitere als TPP bezeichnet), beispielhaft dargestellt gemäß Formel (TPP-I). Die Linker-Gruppe (L) mit dem Antioxidativen Rest (A) sind hier beispielhaft als Kombination von R und Alkylkette dargestellt. Gegenion Z ist hier beispielhaft Bromid.

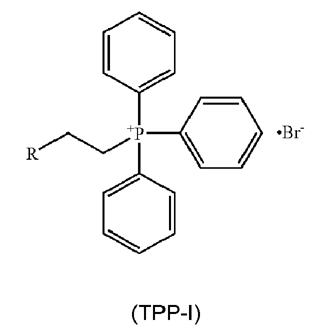

Gemäß einer besonders bevorzugten Ausführungsform der Erfindung ist der antioxidative Rest ausgewählt aus einem Butylhydroxytoluoylrest, Ascorbinsäureresten, insbesondere Ascorbinsäureethern, unsubstituierten oder substituierten 1,4 Hydrochinon- oder 1,4 Benzochinon-Resten, insbesondere ausgewählt aus 2,3-Dimethoxy-5-methyl-1,4-benzochinon-Resten, 1,4-Naphtochinol- und -chinon-Resten, insbesondere den 2-Methyl-1,4-naphthochinol- und -chinon-Resten, 6,7-disubstituierten 2,2-Dialkylchroman- oder -chromen-Resten und unsubstituierten oder substituierten Chroman-6-ol-Resten, insbesondere 2,4,8-Trimethyl-Chroman-6-ol.

Ein bevorzugter antioxidativer Rest (A) wird gebildet aus einem Butylhydroxy-toluolrest (BHT, 2,6-Di-*tert*-butyl-4-methylphenol). BHT ist ein Antioxidans gemäß der Formel BHT-I. Die Anknüpfung des Butylhydroxytoluolrest an die Linkergruppe erfolgt bevorzugt über die Methylgruppe des Toluols.

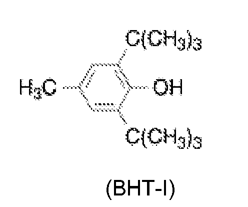

Ein erfindungsgemäß besonders bevorzugter antioxidativer Rest (A) wird gebildet aus einem unsubstituierten oder substituierten 1,4-Hydrochinon- oder 1,4-Benzochinon-Rest. Formel TPP-II zeigt eine entsprechende antioxidative Substanz mit einem bevorzugten antioxidativen Rest (A), der aus einem ggf. substituierten 1,4 Hydrochinon-Rest, sowie einer Linkergruppe (L) mit n Kohlenstoffatomen (Cn) und einer Triphenylphosphonium-gruppe als kationische, lipophile Gruppe (K) gebildet wird.

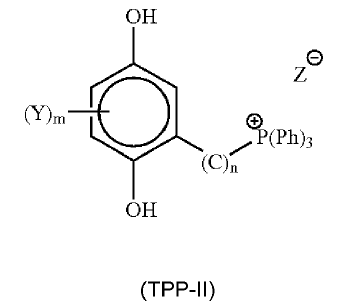

Die Substituenten (Y)ₘ, (mit m = 0, 1, 2 oder 3) am aromatischen System des 1,4-Hydrochinon- bzw. 1,4-Benzochinon-Rests sind unabhängig voneinander bevorzugt ausgewählt aus Alkylresten, insbesondere C₁- bis C₄-Alkylresten, besonders bevorzugt Methyl, Ethyl-, Propyl-, iso-Propyl-, n-Butyl-, 2-Methylpropyl- oder tert.- Butyl-, sowie Alkoxyl-Resten, bevorzugt C₁- bis C₄-Alkoxyl-Resten, insbesondere Methoxy- und Ethoxy-Resten.

Ein besonders bevorzugter antioxidativer Rest (A), der eine substituierte 1,4- Benzochinon- bzw. 1,4-Hydrochinon-Einheit aufweist, ist der 2,3-Dimethoxy-5-methyl-1,4-benzochinon-Rest, bei denen m=3 und Y an 2 und 3-Position Methoxy sowie an 5-Position Methyl ist. An der freien 6-Position des 2,3-Dimethoxy-5-methyl-1,4-benzochinon kann direkt die Linker-Gruppe (L) angeknüpft sein.

2,3-Dimethoxy-5-methyl-1,4-benzochinon ist das Grundgerüst der Ubichinone (UBI-1). Die Ubichinone tragen an der freien 6-Position eine Isoprenoidkette mit n = 6 bis 10. Die Substanz nach Formel UBI-I mit n=0 wird aber ebenfalls als Ubichinon bezeichnet Die entsprechenden Hydrochinone werden auch als Ubichinole bezeichnet. Auch die Isoprenoidketten gemäß Formel UBI-I können als Linker-Gruppe dienen. 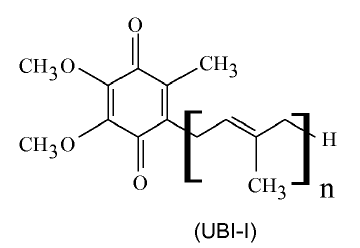 mit n = 6, 7, 8, 9 oder 10. 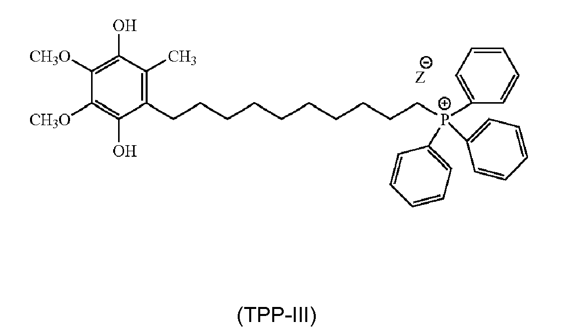 TPP-III stellt eine besonders bevorzugte antioxidative Substanz ("TPP-Q10", MitoQ® erhältlich von Antipodean Pharmaceuticals) mit einem bevorzugten antioxidativen Rest (= 2,3-Dimethoxy-5-methyl-1,4-benzodihydrochinons; reduzierte Form des 2,3-Dimethoxy-5-methyl-1,4-benzochinons) sowie einer bevorzugten Linkergruppe (unverzweigte Alkylkette mit einer Kettenlänge von 10 Kohlenstoffatomen) dar.

Ein anderer ebenfalls bevorzugter antioxidativer Rest (A) ist auswählt aus 6,7-disubstituierten 2,2-Dialkylchromanen oder -chromenen der allgemeinen Formel (DAC-I) oder (DAC-II), 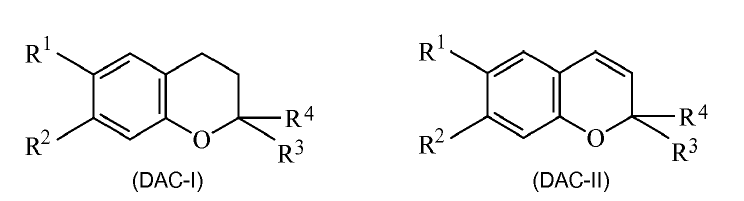 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen. Die Substituenten R¹ und R² sind unabhängig voneinander ausgewählt aus einer OH-Gruppe, einer Methoxy-Gruppe und einer CF₃CH₂O-Gruppe. In einer bevorzugten Ausführungsform sind R¹ und R² unabhängig voneinander ausgewählt aus einer OH-Gruppe und einer Methoxy-Gruppe. In einer besonders bevorzugten Ausführungsform stellen R¹ eine OH-Gruppe und R² eine Methoxy-Gruppe dar.

Einer der Substituenten R³ und R⁴ stellt eine C₁-C₄-Alkylgruppe dar. Unter C₁-C₄-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform ist einer der Substituenten R³ oder R⁴ ausgewählt aus einer Methyl-, Ethyl-, n-Propyl-, Isopropyl- und einer n-Butyl-Gruppe. Besonders bevorzugt stellt R³ bzw. R⁴ eine Methyl- oder Ethylgruppe dar. Der andere der beiden Substituenten R³ bzw. R⁴ ist dabei die Linker-Gruppe (L), welche den antioxidativen Rest (A, der 6,7-disubstituierte 2,2-Dialkylchroman- oder -chromen-Rest) mit der kationischen, lipophilen Gruppe (K) verbindet. Erfindungsgemäß bevorzugt sind die 6,7-disubstituierten 2,2-Dialkylchroman-Reste. Ein erfindungsgemäß außerordentlich bevorzugt verwendeter Rest ist der 2,2-Dimethyl-6-hydroxy-7-methoxy-chroman-Rest mit der systematischen Bezeichnung 3,4-Dihydro-7-methoxy-2,2-dimethyl-2H-1-benzopyran-6-ol.. Ein weiterer besonders bevorzugter antioxidativer Rest (A) wird gebildet aus einem unsubstituierten oder substituierten Chroman-6-ol (3,4-Dihydro-2*H*-1-benzopyran-6-ol). Die Linker-Gruppe (L) ist bevorzugt in 2-Stellung mit dem Chroman-6-ol-Rest verknüpft. Die Substitution des Chroman-6-ols kann unabhängig voneinander ein, zwei oder drei der drei verbliebenen freien Positionen des aromatischen Rings erfolgen. Bevorzugt geeignete Substituenten des Chroman-6-ols sind insbesondere verzweigte oder unverzweigte Alkylketten mit 1 bis 4 Kohlenstoffatomen. Bevorzugt sind dabei insbesondere Methyl-, Ethyl-, Propyl-, iso-Propyl-, Butyl-, 2-Methylpropyl- und tert.-Butyl-Reste, besonders bevorzugt ist Methyl.

Bevorzugt sind in diesem Fall insbesondere Linker-Gruppen mit verzweigten oder unverzweigten Alkylketten mit 1 bis 17, insbesondere 3 bis 16 Kohlenstoffatomen.

Die Formel TPP-IV stellt eine bevorzugte antioxidative Substanz mit einem mit drei Methylgruppen (analog α-Tocopherol, an den Positionen 2,4,8) substituiertem Chroman-6-ol-Rest als antioxidativem Rest (A), einem Ethylrest als Linker-Gruppe (L) und dem Triphenylphosphoniumrest als kationische, lipophile Gruppe (K) dar.

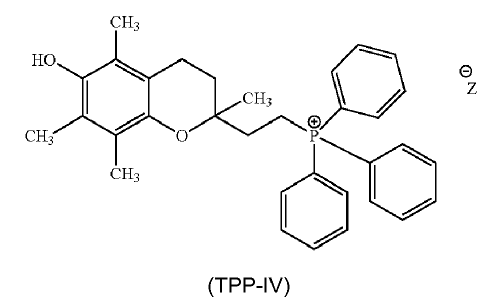

Unter der Bezeichnung "TPP-VitE" ist erfindungsgemäß eine solche Verbindung zu verstehen, die analog zu Formel TPP-IV aufgebaut ist, wobei aber die Linkergruppe zwischen dem dargestellten antioxidativen Rest (A = 2,4,8 Trimethyl-Chroman-6-ol) und der dargestellten kationischen lipophilen Gruppe K=Triphenylphosphonium-Rest, eine Länge von 13 Kohlenstoffatomen, L = -(CH₂)ₙ- mit n = 13, aufweist.

Ist das Chroman-6-ol in 2-Stellung mit einem 4,8,12-Trimethyltridecyl-Rest substituiert, ergibt sich die Grundform der Tocopherole (Formel TOC-I). Der 4,8,12-Trimethyltridecyl-Rest des Tocopherols kann bereits als Linkergruppe verwendet werden, so dass die lipophile, kationische Gruppe (K), bevorzugt TPP, am Ende des Tridecylrestes kovalent gebunden ist. Die unterschiedliche Substitution des aromatischen Ringes von TOC-I (R₁-R₃) mit ein, zwei oder drei Methylgruppen führt zu den verschiedenen bekannten Tocopherolen, α - η (vgl. Tabelle 1), erfindungsgemäß besonders bevorzugt ist das dreifach am aromatischen Ring methylierte Chroman-6-ol, das als alpha-Tocopherol bezeichnet wird.

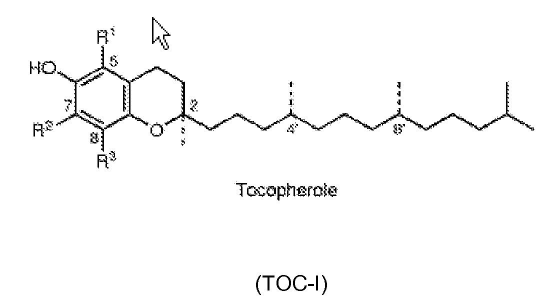

Ebenfalls bevorzugte antioxidative Reste sind die Ringöffnungsprodukte der Tocopherole, die Tocopherolchinone (TOC-II) und die Tocopherolhydrochinone (TOC-III), insbesondere in der dreifach methylierten α-Form (vgl. Tabelle 1).

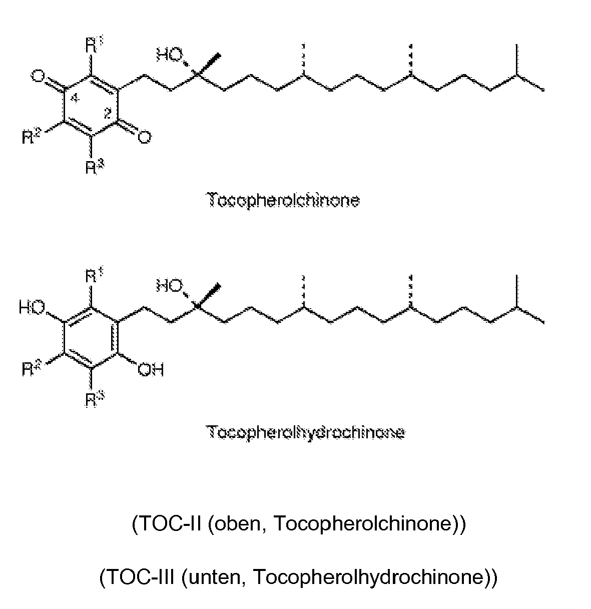

**Tabelle 1: Struktur von Tocopherolen.**

| | **R¹** | **R²** | **R³** | **Konfiguration** | **Summenformel CAS RN** | ***M*ᵣ** |
|---|---|---|---|---|---|---|
| Tocopherole | | | | | | |
| α | CH₃ | CH₃ | CH₃ | 2*R*,4 '*R*,8 *'R* | C₂₉H₅₀O₂ 10191-41-0 59-02-9 | 430,71 |
| β | CH₃ | H | CH₃ | 2*R*,4 '*R*,8 '*R* | C₂₈H₄₈O₂ 148-03-8 16698-35-4 | 416,69 |
| Y | H | CH₃ | CH₃ | 2*R*,4 '*R*,8 '*R* (±)-Form | C₂₈H₄₈O₂ 7616-22-0 54-28-4 7540-59-2 | 416,69 |
| δ | H | H | CH₃ | 2*R*,4 *'R*,8 *'R* | C₂₇H₄₆O₂ 119-13-1 5488-58-4 | 402,66 |
| ζ₂ | CH₃ | CH₃ | H | 2*R*,4 *'R*,8 *'R* | C₂₈H₄₈O₂ 493-35-6 17976-95-3 | 416,69 |
| η | H | CH₃ | H | 2*R*,4 *'R*,8 *'R* | C₂₇H₄₆O₂ 91-86-1 78656-16-3 | 402,64 |
| Tocopherylchinone | | | | | | |
| α | CH₃ | CH₃ | CH₃ | 3 *'R*,7 *'R*,11 '*R* | C₂₉H₅₀O₃ 7559-04-8 | 446,71 |
| | | | | | | |
| Tocopherylhydrochinone | | | | | | |
| α | CH₃ | CH₃ | CH₃ | 3 *'R*,7 *'R*,11 *'R* | C₂₉H₅₂O₃ 14745-36-9 | 448,73 |

Gemäß einer weiteren besonders bevorzugten Ausführungsform der Erfindung ist der antioxidative Rest von der Ascorbinsäure abgeleitet. Die Ascorbinsäure, R-5-[(S)-1,2-Dihydroxyethyl]-3,4-dihydroxy-5*H*-furan-2-on, auch Vitamin C genannt (ASC-I, mit R=H), wirkt antioxidativ. Gleichzeitig ist die Ascorbinsäure physiologisch gut verträglich und daher besonders bevorzugt.

Die Ascorbinsäurereinheit wird bevorzugt über eine der Hydroxylgruppen am Dihydroxyethyl-Rest mit der Linkergruppe kovalent verknüpft. Besonders bevorzugt ist die Verknüpfung über die primäre OH-Gruppe. Die Linkergruppe ist dann als Rest R gemäß der Formel ASC-I kovalent verknüpft. Dies ist erfindungsgemäß bevorzugt als Ascorbinsäurerest zu verstehen.

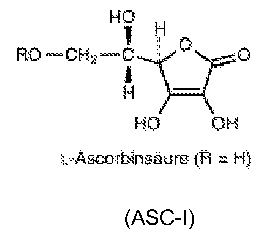

Gemäß einer ganz besonders bevorzugten Ausführungsform der Erfindung ist das R gemäß Formel ASC-I ausgewählt einem Rest der gebildet wird aus eine Linker-Gruppe (L) = -(CH₂)ₙ - mit n= 3, 4, 5,6, 7, 8, 9, 10, 11, 12, 13, 14, 15, 16, 17, die mit einer kationischen, lipophilen Gruppe (K) = Triphenylphosphonium- verknüpft ist.

Unter der Bezeichnung "TPP-VitC" ist erfindungsgemäß eine solche Verbindung zu verstehen, die gemäß Formel ASC-I aufgebaut ist und einen Rest R eine Linkergruppe verknüpft über R als Ether mit L = -(CH₂)ₙ - mit n = 10 und daran geknüpft eine kationische, lipophile Gruppe K=Triphenylphosphonium-Rest aufweist.

Gemäß einer ganz besonders bevorzugten Ausführungsform der Erfindung ist der antioxidative Rest ausgewählt aus einem Ascorbinsäurerest, einem 3,4-Dihydro-2,4,8 Trimethyl-2H-1-benzopyran-6-ol-Rest oder einem 2,2-Dimethyl-6-hydroxy-7-methoxy-chroman-Rest.

Gemäß einer weiteren bevorzugten Ausführungsform der Erfindung ist der antioxidative Rest (A) ausgewählt aus 1,4-Naphthochinonen oder 1,4-Naphthohydrochinonen.

Besonders bevorzugt sind 2-Methylnaphthochinone gemäß Formel (NA-I), wobei die Linkergruppe (L) an der Position 3 kovalent gebunden ist, diese Position ist in der Formel NA-I durch ein R gekennzeichnet.

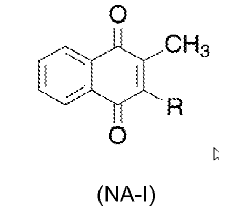

Gemäß einer weiteren bevorzugten Ausführungsform der Erfindung wird die Linker-Gruppe (L) der erfindungsgemäßen antioxidativen Substanz durch eine unsubstituierte oder substituierte Alkyl-, Alkenyl- oder Alkinylkette mit 1 bis 17 Kohlenstoffatomen gebildet.

Gemäß einer weiteren besonders bevorzugten Ausführungsform der Erfindung ist die Linkergruppe eine unsubstituierte oder substituierte Alkylkette.

Gemäß einer besonders bevorzugten Ausführungsform der Erfindung umfasst die substituierte Alkyl-, Alkenyl- oder Alkinylkette einen oder mehrere Substituenten, welche ausgewählt sind aus unsubstituierten oder substituierten Alkyl-, Alkenyl- oder Alkinylseitengruppen, sowie Hydroxyl-, Carboxy- und Amidgruppen.

Besonders bevorzugt sind unsubstituierte, unverzweigte Alkylketten mit 3 bis 15 Kohlenstoffatomen, -(CH₂)ₙ- mit n=3, 4 ,5 ,6 ,7 ,8 ,9 ,10 ,11 ,12 ,13 ,14 oder 15.

Besonders bevorzugte Linker sind solche gemäß Formeln TPP-V bis TPP-VI, dargestellt am Beispiel der erfindungsgemäß bevorzugten Verbindungen mit (K) = Triphenylphosphoniumkation und (A) = 2,3-Dimethoxy-5-methyl-1,4-benzochinon-Rest; TPP-V mit L=-(CH₂)ₙ- mit n=3; TPP-VI mit L=-(CH₂)ₙ- mit n=5; TPP-III mit L=-(CH₂)ₙ- mit n=10 (TPP-Q10, MitoQ^{®}); TPP-VII mit L=-(CH₂)ₙ- mit n=15.

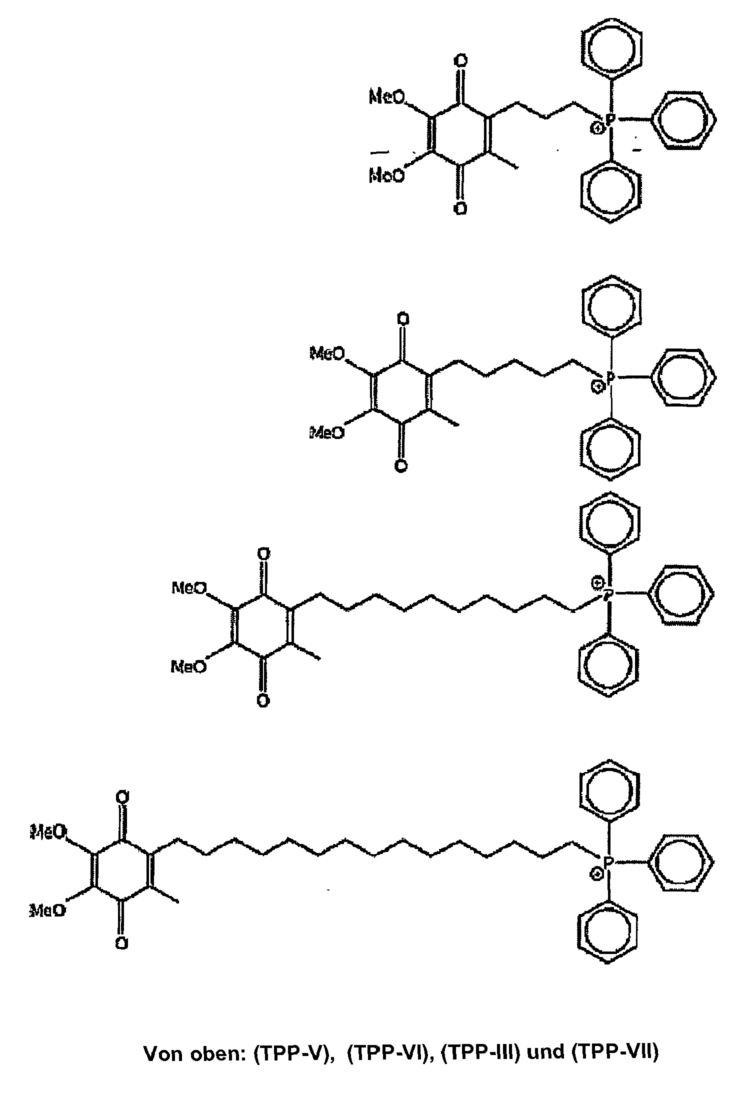

Gemäß einer bevorzugten Ausführungsform der Erfindung ist das kosmetisch und/oder pharmazeutisch akzeptable Gegenion (Z) der antioxidativen Substanz ausgewählt aus Alkylsulfonaten, Arylsulfonaten und Halogenen. Besonders bevorzugt als Gegenion sind insbesondere Benzolsulfonat, Methylbenzyolsulfonat und 2-Naphthalinsulfonat, Methansulfonat, Ethansulfonat, Nitrat und Bromid.

Besonders bevorzugte antioxidative Substanzen sind in der folgenden Tabelle aufgelistet:

| A | L | K | Z |
|---|---|---|---|
| | | Kation. | |
| antioxidativer Rest | (CH2)n- | Einheit | Anion |
| | n= | | |
| 3,4-Dihydro-2,4,8 Trimethyl-2H-1-benzopyran-6-ol- | 1 | TPP | Nitrat |
| 3,4-Dihydro-2,4,8 Trimethyl-2H-1-benzopyran-6-ol- | 2 | TPP | Nitrat |
| 3,4-Dihydro-2,4,8 Trimethyl-2H-1-benzopyran-6-ol- | 3 | TPP | Nitrat |
| 3,4-Dihydro-2,4,8 Trimethyl-2H-1-benzopyran-6-ol- | 4 | TPP | Nitrat |
| 3,4-Dihydro-2,4,8 Trimethyl-2H-1-benzopyran-6-ol- | 5 | TPP | Nitrat |
| 3,4-Dihydro-2,4,8 Trimethyl-2H-1-benzopyran-6-ol- | 6 | TPP | Nitrat |
| 3,4-Dihydro-2,4,8 Trimethyl-2H-1-benzopyran-6-ol- | 7 | TPP | Nitrat |
| 3,4-Dihydro-2,4,8 Trimethyl-2H-1-benzopyran-6-ol- | 8 | TPP | Nitrat |
| 3,4-Dihydro-2,4,8 Trimethyl-2H-1-benzopyran-6-ol- | 9 | TPP | Nitrat |
| 3,4-Dihydro-2,4,8 Trimethyl-2H-1-benzopyran-6-ol- | 10 | TPP | Nitrat |
| 3,4-Dihydro-2,4,8 Trimethyl-2H-1-benzopyran-6-ol- | 11 | TPP | Nitrat |
| 3,4-Dihydro-2,4,8 Trimethyl-2H-1-benzopyran-6-ol- | 12 | TPP | Nitrat |
| 3,4-Dihydro-2,4,8 Trimethyl-2H-1-benzopyran-6-ol- | 13 | TPP | Nitrat |
| 3,4-Dihydro-2,4,8 Trimethyl-2H-1-benzopyran-6-ol- | 14 | TPP | Nitrat |
| 3,4-Dihydro-2,4,8 Trimethyl-2H-1-benzopyran-6-ol- | 15 | TPP | Nitrat |
| | | | |
| 3,4-Dihydro-2,4,8 Trimethyl-2H-1-benzopyran-6-ol- | 1 | TPP | Bromid |
| 3,4-Dihydro-2,4,8 Trimethyl-2H-1-benzopyran-6-ol- | 2 | TPP | Bromid |
| 3,4-Dihydro-2,4,8 Trimethyl-2H-1-benzopyran-6-ol- | 3 | TPP | Bromid |
| 3,4-Dihydro-2,4,8 Trimethyl-2H-1-benzopyran-6-ol- | 4 | TPP | Bromid |
| 3,4-Dihydro-2,4,8 Trimethyl-2H-1-benzopyran-6-ol- | 5 | TPP | Bromid |
| 3,4-Dihydro-2,4,8 Trimethyl-2H-1-benzopyran-6-ol- | 6 | TPP | Bromid |
| 3,4-Dihydro-2,4,8 Trimethyl-2H-1-benzopyran-6-ol- | 7 | TPP | Bromid |
| 3,4-Dihydro-2,4,8 Trimethyl-2H-1-benzopyran-6-ol- | 8 | TPP | Bromid |
| 3,4-Dihydro-2,4,8 Trimethyl-2H-1-benzopyran-6-ol- | 9 | TPP | Bromid |
| 3,4-Dihydro-2,4,8 Trimethyl-2H-1-benzopyran-6-ol- | 10 | TPP | Bromid |
| 3,4-Dihydro-2,4,8 Trimethyl-2H-1-benzopyran-6-ol- | 11 | TPP | Bromid |
| 3,4-Dihydro-2,4,8 Trimethyl-2H-1-benzopyran-6-ol- | 12 | TPP | Bromid |
| 3,4-Dihydro-2,4,8 Trimethyl-2H-1-benzopyran-6-ol- | 13 | TPP | Bromid |
| 3,4-Dihydro-2,4,8 Trimethyl-2H-1-benzopyran-6-ol- | 14 | TPP | Bromid |
| 3,4-Dihydro-2,4,8 Trimethyl-2H-1-benzopyran-6-ol- | 15 | TPP | Bromid |
| 3,4-Dihydro-2,4,8 Trimethyl-2H-1-benzopyran-6-ol- | 14 | TPP | Bromid |
| 3,4-Dihydro-2,4,8 Trimethyl-2H-1-benzopyran-6-ol- | 15 | TPP | Bromid |
| | | | |
| 3,4-Dihydro-2,4,8 Trimethyl-2H-1-benzopyran-6-ol- | 1 | TPP | Methansulfonat |
| 3,4-Dihydro-2,4,8 Trimethyl-2H-1-benzopyran-6-ol- | 2 | TPP | Methansulfonat |
| 3,4-Dihydro-2,4,8 Trimethyl-2H-1-benzopyran-6-ol- | 3 | TPP | Methansulfonat |
| 3,4-Dihydro-2,4,8 Trimethyl-2H-1-benzopyran-6-ol- | 4 | TPP | Methansulfonat |
| 3,4-Dihydro-2,4,8 Trimethyl-2H-1-benzopyran-6-ol- | 5 | TPP | Methansulfonat |
| 3,4-Dihydro-2,4,8 Trimethyl-2H-1-benzopyran-6-ol- | 6 | TPP | Methansulfonat |
| 3,4-Dihydro-2,4,8 Trimethyl-2H-1-benzopyran-6-ol- | 7 | TPP | Methansulfonat |
| 3,4-Dihydro-2,4,8 Trimethyl-2H-1-benzopyran-6-ol- | 8 | TPP | Methansulfonat |
| 3,4-Dihydro-2,4,8 Trimethyl-2H-1-benzopyran-6-ol- | 9 | TPP | Methansulfonat |
| 3,4-Dihydro-2,4,8 Trimethyl-2H-1-benzopyran-6-ol- | 10 | TPP | Methansulfonat |
| 3,4-Dihydro-2,4,8 Trimethyl-2H-1-benzopyran-6-ol- | 11 | TPP | Methansulfonat |
| 3,4-Dihydro-2,4,8 Trimethyl-2H-1-benzopyran-6-ol- | 12 | TPP | Methansulfonat |
| 3,4-Dihydro-2,4,8 Trimethyl-2H-1-benzopyran-6-ol- | 13 | TPP | Methansulfonat |
| 3,4-Dihydro-2,4,8 Trimethyl-2H-1-benzopyran-6-ol- | 14 | TPP | Methansulfonat |
| 3,4-Dihydro-2,4,8 Trimethyl-2H-1-benzopyran-6-ol- | 15 | TPP | Methansulfonat |
| | | | |
| 3,4-Dihydro-2,4,8 Trimethyl-2H-1-benzopyran-6-ol- | 1 | TPP | Ethansulfonat |
| 3,4-Dihydro-2,4,8 Trimethyl-2H-1-benzopyran-6-ol- | 2 | TPP | Ethansulfonat |
| 3,4-Dihydro-2,4,8 Trimethyl-2H-1-benzopyran-6-ol- | 3 | TPP | Ethansulfonat |
| 3,4-Dihydro-2,4,8 Trimethyl-2H-1-benzopyran-6-ol- | 4 | TPP | Ethansulfonat |
| 3,4-Dihydro-2,4,8 Trimethyl-2H-1-benzopyran-6-ol- | 5 | TPP | Ethansulfonat |
| 3,4-Dihydro-2,4,8 Trimethyl-2H-1-benzopyran-6-ol- | 6 | TPP | Ethansulfonat |
| 3,4-Dihydro-2,4,8 Trimethyl-2H-1-benzopyran-6-ol- | 7 | TPP | Ethansulfonat |
| 3,4-Dihydro-2,4,8 Trimethyl-2H-1-benzopyran-6-ol- | 8 | TPP | Ethansulfonat |
| 3,4-Dihydro-2,4,8 Trimethyl-2H-1-benzopyran-6-ol- | 9 | TPP | Ethansulfonat |
| 3,4-Dihydro-2,4,8 Trimethyl-2H-1-benzopyran-6-ol- | 10 | TPP | Ethansulfonat |
| 3,4-Dihydro-2,4,8 Trimethyl-2H-1-benzopyran-6-ol- | 11 | TPP | Ethansulfonat |
| 3,4-Dihydro-2,4,8 Trimethyl-2H-1-benzopyran-6-ol- | 12 | TPP | Ethansulfonat |
| 3,4-Dihydro-2,4,8 Trimethyl-2H-1-benzopyran-6-ol- | 13 | TPP | Ethansulfonat |
| 3,4-Dihydro-2,4,8 Trimethyl-2H-1-benzopyran-6-ol- | 14 | TPP | Ethansulfonat |
| 3,4-Dihydro-2,4,8 Trimethyl-2H-1-benzopyran-6-ol- | 15 | TPP | Ethansulfonat |
| | | | |
| 3,4-Dihydro-7-methoxy-2,2-dimethyl-2H-1-benzopyran-6-ol | 1 | TPP | Nitrat |
| 3,4-Dihydro-7-methoxy-2,2-dimethyl-2H-1-benzopyran-6-ol | 2 | TPP | Nitrat |
| 3,4-Dihydro-7-methoxy-2,2-dimethyl-2H-1-benzopyran-6-ol | 3 | TPP | Nitrat |
| 3,4-Dihydro-7-methoxy-2,2-dimethyl-2H-1-benzopyran-6-ol | 4 | TPP | Nitrat |
| 3,4-Dihydro-7-methoxy-2,2-dimethyl-2H-1-benzopyran-6-ol | 5 | TPP | Nitrat |
| 3,4-Dihydro-7-methoxy-2,2-dimethyl-2H-1-benzopyran-6-ol | 6 | TPP | Nitrat |
| 3,4-Dihydro-7-methoxy-2,2-dimethyl-2H-1-benzopyran-6-ol | 7 | TPP | Nitrat |
| 3,4-Dihydro-7-methoxy-2,2-dimethyl-2H-1-benzopyran-6-ol | 8 | TPP | Nitrat |
| 3,4-Dihydro-7-methoxy-2,2-dimethyl-2H-1-benzopyran-6-ol | 9 | TPP | Nitrat |
| 3,4-Dihydro-7-methoxy-2,2-dimethyl-2H-1-benzopyran-6-ol | 10 | TPP | Nitrat |
| 3,4-Dihydro-7-methoxy-2,2-dimethyl-2H-1-benzopyran-6-ol | 11 | TPP | Nitrat |
| 3,4-Dihydro-7-methoxy-2,2-dimethyl-2H-1-benzopyran-6-ol | 12 | TPP | Nitrat |
| 3,4-Dihydro-7-methoxy-2,2-dimethyl-2H-1-benzopyran-6-ol | 13 | TPP | Nitrat |
| 3,4-Dihydro-7-methoxy-2,2-dimethyl-2H-1-benzopyran-6-ol | 14 | TPP | Nitrat |
| 3,4-Dihydro-7-methoxy-2,2-dimethyl-2H-1-benzopyran-6-ol | 15 | TPP | Nitrat |
| | | | |
| 3,4-Dihydro-7-methoxy-2,2-dimethyl-2H-1-benzopyran-6-ol | 1 | TPP | Bromid |
| 3,4-Dihydro-7-methoxy-2,2-dimethyl-2H-1-benzopyran-6-ol | 2 | TPP | Bromid |
| 3,4-Dihydro-7-methoxy-2,2-dimethyl-2H-1-benzopyran-6-ol | 3 | TPP | Bromid |
| 3,4-Dihydro-7-methoxy-2,2-dimethyl-2H-1-benzopyran-6-ol | 4 | TPP | Bromid |
| 3,4-Dihydro-7-methoxy-2,2-dimethyl-2H-1-benzopyran-6-ol | 5 | TPP | Bromid |
| 3,4-Dihydro-7-methoxy-2,2-dimethyl-2H-1-benzopyran-6-ol | 6 | TPP | Bromid |
| 3,4-Dihydro-7-methoxy-2,2-dimethyl-2H-1-benzopyran-6-ol | 7 | TPP | Bromid |
| 3,4-Dihydro-7-methoxy-2,2-dimethyl-2H-1-benzopyran-6-ol | 8 | TPP | Bromid |
| 3,4-Dihydro-7-methoxy-2,2-dimethyl-2H-1-benzopyran-6-ol | 9 | TPP | Bromid |
| 3,4-Dihydro-7-methoxy-2,2-dimethyl-2H-1-benzopyran-6-ol | 10 | TPP | Bromid |
| 3,4-Dihydro-7-methoxy-2,2-dimethyl-2H-1-benzopyran-6-ol | 11 | TPP | Bromid |
| 3,4-Dihydro-7-methoxy-2,2-dimethyl-2H-1-benzopyran-6-ol | 12 | TPP | Bromid |
| 3,4-Dihydro-7-methoxy-2,2-dimethyl-2H-1-benzopyran-6-ol | 13 | TPP | Bromid |
| 3,4-Dihydro-7-methoxy-2,2-dimethyl-2H-1-benzopyran-6-ol | 14 | TPP | Bromid |
| 3,4-Dihydro-7-methoxy-2,2-dimethyl-2H-1-benzopyran-6-ol | 15 | TPP | Bromid |
| | | | |
| 3,4-Dihydro-7-methoxy-2,2-dimethyl-2H-1-benzopyran-6-ol | 1 | TPP | Methansulfonat |
| 3,4-Dihydro-7-methoxy-2,2-dimethyl-2H-1-benzopyran-6-ol | 2 | TPP | Methansulfonat |
| 3,4-Dihydro-7-methoxy-2,2-dimethyl-2H-1-benzopyran-6-ol | 3 | TPP | Methansulfonat |
| 3,4-Dihydro-7-methoxy-2,2-dimethyl-2H-1-benzopyran-6-ol | 4 | TPP | Methansulfonat |
| 3,4-Dihydro-7-methoxy-2,2-dimethyl-2H-1-benzopyran-6-ol | 5 | TPP | Methansulfonat |
| 3,4-Dihydro-7-methoxy-2,2-dimethyl-2H-1-benzopyran-6-ol | 6 | TPP | Methansulfonat |
| 3,4-Dihydro-7-methoxy-2,2-dimethyl-2H-1-benzopyran-6-ol | 7 | TPP | Methansulfonat |
| 3,4-Dihydro-7-methoxy-2,2-dimethyl-2H-1-benzopyran-6-ol | 8 | TPP | Methansulfonat |
| 3,4-Dihydro-7-methoxy-2,2-dimethyl-2H-1-benzopyran-6-ol | 9 | TPP | Methansulfonat |
| 3,4-Dihydro-7-methoxy-2,2-dimethyl-2H-1-benzopyran-6-ol | 10 | TPP | Methansulfonat |
| 3,4-Dihydro-7-methoxy-2,2-dimethyl-2H-1-benzopyran-6-ol | 11 | TPP | Methansulfonat |
| 3,4-Dihydro-7-methoxy-2,2-dimethyl-2H-1-benzopyran-6-ol | 12 | TPP | Methansulfonat |
| 3,4-Dihydro-7-methoxy-2,2-dimethyl-2H-1-benzopyran-6-ol | 13 | TPP | Methansulfonat |
| 3,4-Dihydro-7-methoxy-2,2-dimethyl-2H-1-benzopyran-6-ol | 14 | TPP | Methansulfonat |
| 3,4-Dihydro-7-methoxy-2,2-dimethyl-2H-1-benzopyran-6-ol | 15 | TPP | Methansulfonat |
| | | | |
| 3,4-Dihydro-7-methoxy-2,2-dimethyl-2H-1-benzopyran-6-ol | 1 | TPP | Ethansulfonat |
| 3,4-Dihydro-7-methoxy-2,2-dimethyl-2H-1-benzopyran-6-ol | 2 | TPP | Ethansulfonat |
| 3,4-Dihydro-7-methoxy-2,2-dimethyl-2H-1-benzopyran-6-ol | 3 | TPP | Ethansulfonat |
| 3,4-Dihydro-7-methoxy-2,2-dimethyl-2H-1-benzopyran-6-ol | 4 | TPP | Ethansulfonat |
| 3,4-Dihydro-7-methoxy-2,2-dimethyl-2H-1-benzopyran-6-ol | 5 | TPP | Ethansulfonat |
| 3,4-Dihydro-7-methoxy-2,2-dimethyl-2H-1-benzopyran-6-ol | 6 | TPP | Ethansulfonat |
| 3,4-Dihydro-7-methoxy-2,2-dimethyl-2H-1-benzopyran-6-ol | 7 | TPP | Ethansulfonat |
| 3,4-Dihydro-7-methoxy-2,2-dimethyl-2H-1-benzopyran-6-ol | 8 | TPP | Ethansulfonat |
| 3,4-Dihydro-7-methoxy-2,2-dimethyl-2H-1-benzopyran-6-ol | 9 | TPP | Ethansulfonat |
| 3,4-Dihydro-7-methoxy-2,2-dimethyl-2H-1-benzopyran-6-ol | 10 | TPP | Ethansulfonat |
| 3,4-Dihydro-7-methoxy-2,2-dimethyl-2H-1-benzopyran-6-ol | 11 | TPP | Ethansulfonat |
| 3,4-Dihydro-7-methoxy-2,2-dimethyl-2H-1-benzopyran-6-ol | 12 | TPP | Ethansulfonat |
| 3,4-Dihydro-7-methoxy-2,2-dimethyl-2H-1-benzopyran-6-ol | 13 | TPP | Ethansulfonat |
| 3,4-Dihydro-7-methoxy-2,2-dimethyl-2H-1-benzopyran-6-ol | 14 | TPP | Ethansulfonat |
| 3,4-Dihydro-7-methoxy-2,2-dimethyl-2H-1-benzopyran-6-ol | 15 | TPP | Ethansulfonat |

| | | | |
|---|---|---|---|
| TPP= unsubstituiertes Triphenylphosphoniumkation | | | |

Gemäß einer besonders bevorzugten Ausführungsform der Erfindung ist die antioxidative Substanz (A)-(L)-(K)⁺ (Z)⁻ in einer Konzentration von 0,00001 bis 10 Gew.-%, bevorzugt zu 0,0001 bis 1 Gew.-%, weiter bevorzugt 0,001 - 0,5 Gew.-%, weiter bevorzugt 0,01 - 0,1 Gew.-%, jeweils bezogen auf das Gesamtgewicht der Zusammensetzung, in der erfindungsgemäßen Zusammensetzung enthalten.

Gemäß einer bevorzugten Ausführungsform der Erfindung enthält die erfindungsgemäße Zusammensetzung, enthaltend eine antioxidative Substanz (A)-(L)-(K)⁺ (Z)⁻ und 0,0001 bis 99 Gew.-% mindestens eines Tensids, mindestens einen zusätzlichen Wirkstoff, ausgewählt aus Aporphin-Alkaloiden, Purin und/oder Purinderivaten, natürlichen Betainverbindungen, alkyl- oder hydroxyalkylsubstituierten Harnstoffverbindungen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe.

Überraschend wurde festgestellt, dass die Wirkung der erfindungsgemäßen Zusammensetzung in unerwarteter Weise durch ein Aporphin-Alkaloid gesteigert werden kann. Ohne an diese Theorie gebunden sein zu wollen, wird vermutet, dass der Zusatz von mindestens einem Aporphin-Alkaloid zu einer optimierten Regulierung der Elektrolytversorgung auf zellulärer Ebene führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird.

Erfindungsgemäß bevorzugt zu verwendende Aporphin-Alkaloide sind ausgewählt ist aus Verbindungen der allgemeinen Struktur (APO-ALK-I), 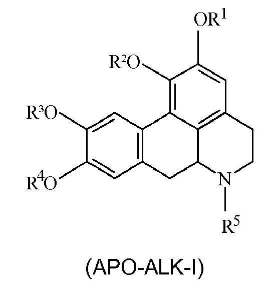 in der die Reste R¹, R², R³, R⁴ und R⁵, die gleich oder verschieden sein können, ausgewählt sind aus einem Wasserstoffatom, einem Halogenatom, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe, einer Aryl-C₁-C₆-alkylgruppe, einer Acylgruppe, einer Sulfonylgruppe oder einem Zucker, und den kosmetisch verträglichen Salzen von (APO-ALK-I) mit einer Säure, in Form aller 15 optischen Isomere, in isomerenreiner Form oder als Mischung optischer Isomere.

Beispiele für die als Substituenten in den erfindungsgemäß kombinierten Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugt zu verwendende C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt.

Als Zuckerrest können beliebige Mono- oder Oligo-, insbesondere Disaccharide, eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Besonders bevorzugte Aporphin-Alkaloide sind ausgewählt aus Verbindungen der allgemeinen Struktur (APO-ALK-I), in denen R¹ = R² = R³ = R⁴ = R⁵ = CH₃ ist. Diese Verbindung wird auch als 1,2,9,10-Tetramethoxyaporphin bezeichnet.

Besonders bevorzugt erfindungsgemäße Zusammensetzungen enthalten mindestens ein Aporphin-Alkaloid in engeren Mengenbereichen. Hier sind erfindungsgemäß bevorzugte Zusammensetzungen, insbesondere kosmetische Zusammensetzungen, dadurch gekennzeichnet, dass sie - bezogen auf ihr Gewicht - eine Gesamtmenge von 0,0001 - 1 Gew.-%, vorzugsweise 0,001 - 0,5 Gew.-%, besonders bevorzugt 0,002 - 0,1 Gew.-% und insbesondere 0,005 - 0,01 Gew.-% mindestens eines Aporphin-Alkaloids enthalten.

Erfindungsgemäß besonders bevorzugte Zusammensetzungen enthalten TPP-VitE und 1,2,9,10-Tetramethoxyaporphin. Weitere erfindungsgemäß besonders bevorzugte Zusammensetzungen enthalten TPP-VitC und 1,2,9,10-Tetramethoxyaporphin. Weitere erfindungsgemäß besonders bevorzugte Zusammensetzungen enthalten TPP-MitQ und 1,2,9,10-Tetramethoxyaporphin.

Ein weiterer Gegenstand der vorliegenden Erfindung ist eine Zusammensetzung, enthaltend eine antioxidative Substanz und mindestens ein Tensid, in Kombination mit mindestens einem Wirkstoff enthalten, der ausgewählt ist aus Purin (7*H-*Imidazo[4,5-*d*]pyrimidin) und den Derivaten des Purins. Überraschend wurde festgestellt, dass die Wirkung der erfindungsgemäßen Zusammensetzung in unerwarteter Weise durch Purin und/oder Purinderivat(e) gesteigert werden kann. Ohne an diese Theorie gebinden sein zu wollen, wird vermutet, dass der Zusatz von Purin und/oder mindestens einem Purinderivat zu einer optimierten Regulierung der Nährstoffversorgung, insbesondere der Lipidversorgung, auf zellulärer Ebene führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird. Besonders bevorzugte erfindungsgemäße kosmetische oder dermatologische Zusammensetzungen enthalten Purin und/oder Purinderivat(e) - bezogen auf ihr Gewicht - in einer Gesamtmenge von 0,001 - 2,5 Gew.-%, bevorzugt 0,01 - 1 Gew.-%, besonders bevorzugt 0,1 - 0,8 Gew.-% und insbesondere 0,2 - 0,5 Gew.-%. Unter Purin, den Purinen und den Purinderivaten sind einige Vertreter erfindungsgemäß besonders bevorzugt. Erfindungsgemäß bevorzugte Zusammensetzungen sind dadurch gekennzeichnet, dass sie Purin und/oder Purinderivat(e) der Formel (PUR-I) enthalten, 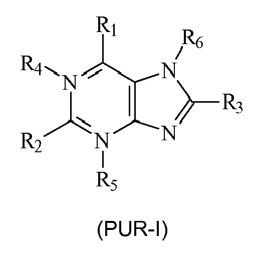 in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁- bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

Erfindungsgemäß besonders bevorzugt zu verwendende Kombinationen sind **dadurch gekennzeichnet, dass** sie Purin und/oder Purinderivat(e) der Formel (PUR-I) enthalten, in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, -CH₃ und -CH₂-CH₃, wobei folgende Verbindungen außerordentlich bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ R⁴ = H, R⁵ = R⁶ = CH₃)
- Theophyllin (R¹ = R² = OH, R³ = H, R⁴ = CH₃, R⁵ = CH₃, R⁶ = H).

Verbindungen der allgemeinen Formel (PUR-I), in denen R¹ und/oder R² einer OH-Gruppe entsprechen, existieren in tautomeren Verbindungen, insbesondere solchen der allgemeinen Formel (PUR-II), ausgehend von (PUR-I) mit R¹ = R² = OH: 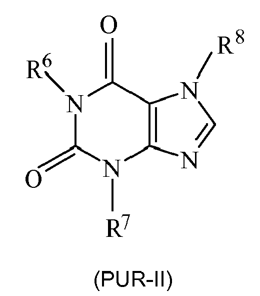 wobei in der Formel (PUR-II) die Reste R⁶, R⁷ und R⁸, die gleich oder verschieden sein können, ausgewählt sind aus einem Wasserstoffatom, einem C₁- bis C₄-Alkylrest, einem C₁- bis C₄-Mono-hydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe, wobei Coffein (R⁶ = R⁷ = R⁸ = CH₃), Theobromin (R⁶ = H, R⁷ = R⁸ = CH₃) und Theophyllin (R⁶ = R⁷ = CH₃, R⁸ = H) außerordentlich bevorzugt sind.

Erfindungsgemäß besonders bevorzugte Zusammensetzungen enthalten TPP-VitC und Coffein. Weitere erfindungsgemäß besonders bevorzugte Zusammensetzungen enthalten TPP-VitC und Theobromin. Weitere erfindungsgemäß besonders bevorzugte Zusammensetzungen enthalten TPP-VitC und Theophyllin. Weitere erfindungsgemäß besonders bevorzugte Zusammensetzungen enthalten TPP-MitQ und Coffein. Weitere erfindungsgemäß besonders bevorzugte Zusammensetzungen enthalten TPP-MitQ und Theobromin. Weitere erfindungsgemäß besonders bevorzugte Zusammensetzungen enthalten TPP-MitQ und Theophyllin. Weitere erfindungsgemäß besonders bevorzugte Zusammensetzungen enthalten TPP-VitE und Coffein. Weitere erfindungsgemäß besonders bevorzugte Zusammensetzungen enthalten TPP-VitE und Theobromin. Weitere erfindungsgemäß besonders bevorzugte Zusammensetzungen enthalten TPP-VitE und Theophyllin. Weitere erfindungsgemäß besonders bevorzugte Zusammensetzungen enthalten neben der antioxidativen Substanz (A)-(L)-(K)⁺ (Z)⁻und 0,0001 - 99 Gew.-% mindestens eines Tensids weiterhin mindestens eine natürliche Betainverbindung.

Überraschend wurde festgestellt, dass die Wirkung der erfindungsgemäßen Zusammensetzung in unerwarteter Weise durch eine natürliche Betainverbindung gesteigert werden kann. Ohne an diese Theorie gebunden sein zu wollen, wird vermutet, dass der Zusatz von mindestens einer natürlichen Betainverbindung zu einer optimierten Regulierung der Nährstoffversorgung auf zellulärer Ebene und/oder innerhalb der extracellulären Matrix (ECM) führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird.

Erfindungsgemäß bevorzugt zu verwendende natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻), Carnitin (Me₃N⁺-CH₂-CHOH-CH₂-COO⁻), jeweils mit Me = Methyl, und Ergothionein = 2-Mercapto-N^{α}N^{α}N^{α}-trimethyl-L-histidinium-Betain der Formel (BETA-II)

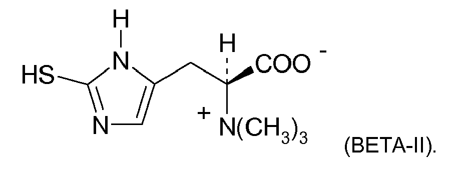

Die Herkunft der eingesetzten natürlichen Betainverbindung kann erfindungsgemäß durchaus synthetisch sein. Weiterhin können auch Salze und/oder Ester der natürlichen Betainverbindungen erfindungsgemäß bevorzugt eingesetzt werden. Ein derartiges besonders bevorzugtes Derivat ist Carnitintartrat. Bevorzugte erfindungsgemäße Zusammensetzungen enthalten zusätzlich mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte Zusammensetzung.

Erfindungsgemäß besonders bevorzugte Zusammensetzungen enthalten TPP-VitC oder TPP-VitE oder TPP-MitQ® und Betain. Eine andere ebenfalls erfindungsgemäß besonders bevorzugt zu verwendende Kombination ist eine antioxidative Substanz, ausgewählt aus TPP-VitC oder TPP-VitE oder TPP-MitQ^{®}, in Kombination mit Carnitin, ebenso eine antioxidative Substanz, ausgewählt aus TPP-VitC oder TPP-VitE oder TPP-MitQ^{®}, in Kombination mit Carnitintartrat. Weitere erfindungsgemäß besonders bevorzugte Zusammensetzungen enthalten eine antioxidative Substanz, ausgewählt aus TPP-VitC oder TPP-VitE oder TPP-MitQ^{®}, in Kombination mit Ergothionein. Weitere erfindungsgemäß besonders bevorzugte Zusammensetzungen enthalten neben der antioxidativen Substanz (A)-(L)-(K)⁺ (Z)⁻und 0,0001 - 99 Gew.-% mindestens eines Tensids weiterhin mindestens eine Substanz, die ausgewählt ist aus Harnstoff und alkyl- oder hydroxyalkylsubstituierten Harnstoffen. Überraschend wurde festgestellt, dass die Wirkung der erfindungsgemäßen Zusammensetzung in unerwarteter Weise durch Substanzen, ausgewählt aus Harnstoff und alkyl- oder hydroxyalkylsubstituierten Harnstoffen, gesteigert werden kann. Ohne an diese Theorie gebunden sein zu wollen, wird vermutet, dass der Zusatz von mindestens einer Substanz, ausgewählt aus Harnstoff und alkyl- oder hydroxyalkylsubstituierten Harnstoffen, zu einer optimierten Regulierung der Nährstoffversorgung innerhalb der extracellulären Matrix (ECM) führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird. Erfindungsgemäß bevorzugt zu verwendende alkyl- oder hydroxyalkylsubstituierte Harnstoffverbindungen weisen die allgemeine Formel (UREA-I) auf, 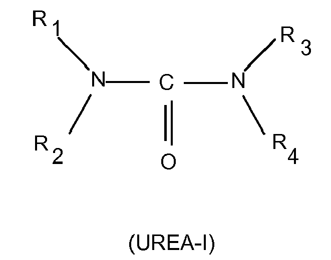 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Erfindungsgemäß besonders bevorzugte alkyl- oder hydroxyalkylsubstituierte Harnstoffverbindungen der allgemeinen Formel (UREA-I) sind ausgewählt aus Verbindungen, bei denen jeweils mindestens einer der Reste R₁ und R₂ bzw. R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Besonders bevorzugte C₂-C₆-Hydroxyalkyl-Gruppen, die mit 1 bis 5 Hydroxylgruppen oder C₁-C₄-Hydroxyalkyl-Gruppen substituiert sind, sind ausgewählt aus 2-Hydroxyethyl-, 3-Hydroxypropyl-, 2-Hydroxypropyl, 2-Hydroxyisopropyl- und 4-Hydroxybutyl-Gruppen, wobei die 2-Hydroxyethyl-Gruppe außerordentlich bevorzugt ist. Ebenfalls außerordentlich bevorzugt ist Bis-N,N'-(2-Hydroxyethyl)-harnstoff. Harnstoff selbst ist ebenfalls besonders bevorzugt. Weiterhin bevorzugt ist es, Mischungen von Harnstoff und alkyl- oder hydroxyalkylsubstituierten Harnstoffverbindungen einzusetzen. Die handelsüblich erhältlichen alkyl- oder hydroxyalkylsubstituierten Harnstoff-Zubereitungen enthalten meistens als Neben- oder Abbauprodukt unsubstituierten Harnstoff. Daher sind erfindungsgemäß auch Kombinationen aus Harnstoff und mindestens einer Substanz, ausgewählt aus alkyl- oder hydroxyalkylsubstituierten Harnstoffen, bevorzugt. Außerordentlich bevorzugt sind Mischungen von Harnstoff und Bis-N,N'-(2-Hydroxyethyl)-harnstoff. Bevorzugte erfindungsgemäß einzusetzende Zusammensetzungen enthalten mindestens eine Substanz, ausgewählt aus Harnstoff und alkyl- oder hydroxyalkylsubstituierten Harnstoffen, in einer Gesamtmenge von 0,01 bis 10 Gew.-%, bevorzugt 0,1 bis 5 Gew.-%, besonders bevorzugt 0,2 bis 3 Gew.-% und außerordentlich bevorzugt 0,5 - 1 Gew.-%, jeweils bezogen auf die gesamte Zusammensetzung.

Eine erfindungsgemäß besonders bevorzugt zu verwendende Kombination ist Bis-N,N'-(2-Hydroxyethyl)-harnstoff und eine antioxidative Substanz, ausgewählt aus TPP-VitC oder TPP-VitE oder TPP-MitQ^{®}. Weitere erfindungsgemäß besonders bevorzugte Zusammensetzungen enthalten Harnstoff und eine antioxidative Substanz, ausgewählt aus TPP-VitC oder TPP-VitE oder TPP-MitQ^{®}. Weitere erfindungsgemäß besonders bevorzugte Zusammensetzungen enthalten Harnstoff und Bis-N,N'-(2-Hydroxyethyl)-harnstoff sowie eine antioxidative Substanz, ausgewählt aus TPP-VitC oder TPP-VitE oder TPP-MitQ^{®}.

Ein weiterer Gegenstand der vorliegenden Erfindung ist eine Zusammensetzung, enthaltend eine antioxidative Substanz und mindestens ein Tensid, in Kombination mit mindestens einer Substanz, die ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe.

Überraschend wurde festgestellt, dass die Wirkung der erfindungsgemäßen Zusammensetzungin unerwarteter Weise durch Substanzen, ausgewählt aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe, gesteigert werden kann. Ohne an diese Theorie gebunden sein zu wollen, wird vermutet, dass der Zusatz von mindestens einer Substanz, ausgewählt aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe, zu einer optimierten Regulierung der Nährstoffversorgung auf zellulärer Ebene und/oder zu einer optimierten Regulierung der Nährstoffversorgung innerhalb der extracellulären Matrix (ECM) und/oder zu einer Stimulierung der Aufbauprozesse der extracellulären Matrix (ECM) führt, wodurch wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird.

Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.

Der C₂ - C₂₄-Acylrest, mit dem die genannten Aminosäuren an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin.

Die physiologisch verträglichen Salze der erfindungsgemäß bevorzugten Aminosäuren oder Aminosäurederivate sind bevorzugt ausgewählt aus den Ammonium-, Alkalimetall-, Magnesium-, Calcium-, Aluminium-, Zink- und Mangan-Salzen. Besonders bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2-15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen.

Erfindungsgemäß bevorzugte, gegebenenfalls N-acylierte und/oder veresterte Dipeptide sind Acetyl-Citrullyl-Arginin (INCl:Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester, Carnosin (β-Ala-His) und N-Palmitoyl-Pro-Arg. Erfindungsgemäß bevorzugte, gegebenenfalls N-acylierte und/oder veresterte Tripeptide sind Gly-His-Lys und N-Palmitoyl-Gly-His-Lys. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCl-Bezeichnung: Tripeptide-3) sowie dessen Derivat N-Myristoyl-Gly-His-Arg; weitere erfindungsgemäß bevorzugt zu verwendende Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, Lys-Pro-Val, Tyr-Tyr-Val, Tyr-Val-Tyr, Val-Tyr-Val (Tripeptide-2), Tripeptide-4, His-Ala-Orn, Gly-Asp-Ser, N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im Folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können. Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile). Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin. Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin. Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt. Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, weiterhin Glu-Glu-Met-Gln-Arg-Arg, weiterhin Acetyl-Hexapeptide-3, Hexapeptide-4, Hexapeptide-5, Myristoyl Hexapeptide-5, Myristoyl Hexapeptide-6, Hexapeptide-8, Myristoyl Hexapeptide-8, Hexapeptide-9 und Hexapeptide-10, Ala-Arg-His-Leu-Phe-Trp (Hexapeptide-1), Acetyl Hexapeptide-1, Acetyl Glutamyl Hexapeptide-1, Hexapeptide-2, Ala-Asp-Leu-Lys-Pro-Thr (Hexapeptide-3), Val-Val-Arg-Pro-Pro-Pro, Hexapeptide-4, Hexapeptide-5, Myristoyl Hexapeptide-5, Myristoyl Hexapeptide-6, Ala-Arg-His-Methylnorleucin-Homophenylalanin-Trp (Hexapeptide-7), Hexapeptide-8, Myristoyl Hexapeptide-8, Hexapeptide-9, Hexapeptide-10 und Hexapeptide-11. Ein erfindungsgemäß bevorzugtes Pentadecapeptid ist z. B. Pentadecapeptide-1. Ein weiteres bevorzugtes Aminosäureoligomer ist das Peptidderivat L-Glutamylaminoethyl-indol. Ein erfindungsgemäß besonders bevorzugter Aminosäureoligomer-Wirkstoff ist die Kombination aus N-Palmitoyl-Gly-His-Lys und N-Palmitoyl-Gly-Gln-Pro-Arg.

Ein weiterer erfindungsgemäß bevorzugter Wirkstoff, der in der erfindungsgemäßen Zusammensetzung eine unerwartete Wirkungssteigerung zeigt, ist ausgewählt aus Polymeren der Aminosäuren und/oder der N-C₂-C₂₄-Acylaminosäuren.

Erfindungsgemäß kombinierte Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. Ebenfalls bevorzugt ist der Einsatz von Acylderivaten der Proteinhydrolysate, z. B. in Form ihrer Fettsäure-Kondensationsprodukte. Erfindungsgemäß bevorzugt sind auch kationisierte Proteinhydrolysate.

In einer weiteren bevorzugten Ausführungsform sind die erfindungsgemäß kombinierten Polymeren der Aminosäuren ausgewählt aus DNA-Reparaturenzymen.

Erfindungsgemäß bevorzugt zu verwendende DNA-Reparaturenzyme sind Photolyase und T4 Endonuclease V, letztere im Weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen. Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome^{™}, liposomenverkapselte T4N5 z. B. unter der Bezeichnung Ultrasome^{™} von der Firma AGI Dermatics, USA, erhältlich. Erfindungsgemäß besonders bevorzugte Zusammensetzungen enthalten zusätzlich mindestens eines der Handelsprodukte Photosomes^{™} oder Ultrasomes^{™} in Gesamtmengen von 0,1 - 10 Gew.-%, bevorzugt 0,5 - 5,0 Gew.-% und besonders bevorzugt 1,0 - 4,0 Gew.-%, bezogen auf die gesamte erfindungsgemäße Zusammensetzung. Weitere erfindungsgemäß besonders bevorzugte Zusammensetzungen enthalten das Enzym Photolyase in einer Gesamtmenge von 0,0001 - 0,1 Gew.-%, bevorzugt 0,001 - 0,05 Gew.-%, besonders bevorzugt 0,002 - 0,02 Gew.-% und außerordentlich bevorzugt 0,005 - 0,01 Gew.-%, jeweils bezogen auf die gesamte erfindungsgemäße Zusammensetzung.

Weitere erfindungsgemäß besonders bevorzugte Zusammensetzungen enthalten das Enzym T4 Endonuclease V in einer Gesamtmenge von 0,0001 - 0,1 Gew.-%, bevorzugt 0,001 - 0,05 Gew.-%, besonders bevorzugt 0,002 - 0,02 Gew.-% und außerordentlich bevorzugt 0,005 - 0,01 Gew.-%, jeweils bezogen auf die gesamte erfindungsgemäße Zusammensetzung.

Erfindungsgemäß besonders bevorzugte Zusammensetzungen enthalten Photolyase und eine antioxidativen Substanz, ausgewählt aus TPP-VitC oder TPP-VitE oder TPP-MitQ^{®}. Weitere erfindungsgemäß besonders bevorzugte Zusammensetzungen enthalten T4 Endonuclease V und eine antioxidative Substanz, ausgewählt aus TPP-VitC oder TPP-VitE oder TPP-MitQ^{®}. Weitere erfindungsgemäß besonders bevorzugte Zusammensetzungen enthalten Photolyase und T4 Endonuclease V und eine antioxidative Substanz, ausgewählt aus TPP-VitC oder TPP-VitE oder TPP-MitQ^{®}.

Erfindungsgemäß besonders bevorzugte Zusammensetzungen sind dadurch gekennzeichnet, dass sie zusätzlich mindestens einen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, in einer Gesamtmenge von 0,00001 - 2 Gew.-%, bevorzugt 0,0001 - 1 Gew.-%, besonders bevorzugt 0,001 - 0,1 Gew.-% und außerordentlich bevorzugt 0,01 - 0,05 Gew.-%, jeweils bezogen auf den Gehalt an Aktivsubstanz in der gesamten Zusammensetzung, enthalten.

Erfindungsgemäß besonders bevorzugte Zusammensetzungen enthalten eine antioxidative Substanz, ausgewählt aus TPP-VitC oder TPP-VitE oder TPP-MitQ^{®}, und mindestens ein Peptid, ausgewählt aus N-Palmitoyl-Gly-His-Lys und/oder N-Palmitoyl-Gly-Gln-Pro-Arg und/oder N-Palmitoyl-Lys-Thr-Thr-Lys-Ser.

Es kann erfindungsgemäß besonders bevorzugt sein, dass die als Wirkstoff eingesetzten Aminosäuren und Peptide zur Verbesserung der Penetration in die Haut mit mindestens einer, bevorzugt linearen, C₂-C₂₂-Fettsäure N-acyliert und/oder verestert sind. Zur N-Acylierung und/oder Veresterung besonders bevorzugt sind C₈-C₁₈-Fettsäuren, ganz besonders bevorzugt sind Myristinsäure (C₁₄) und Palmitinsäure (C₁₆). Besonders bevorzugt ist weiterhin, dass alle verwendeten Aminosäuren und Peptide derartig N-acyliert und/oder verestert sind. Ebenfalls bevorzugt ist die Substitution der Aminosäuren und Peptide mit einer Benzyloxycarbonylgruppe an der terminalen Aminogruppe.

Ein weiterer erfindungsgemäß bevorzugter Wirkstoff, der in Gegenwart der erfindungsgemäßen Zusammensetzung, eine unerwartete Wirkungssteigerung zeigt, ist ausgewählt aus Polysacchariden. Überraschend wurde festgestellt, dass die Wirkung der erfindungsgemäßen Zusammensetzung, in unerwarteter Weise durch Polysaccharide, insbesondere durch Polysaccharide, die mindestens einen Desoxyzucker-Baustein, insbesondere Fucose und/oder Rhamnose, enthalten, und/oder durch Glycosaminoglycane und/oder durch Glucane gesteigert werden kann. Ohne an diese Theorie gebunden sein zu wollen, wird vermutet, dass der Zusatz von mindestens einem Polysaccharid zu einer optimierten Regulierung des Wasserhaushalts innerhalb der extracellulären Matrix (ECM) und/oder zu einer optimierten Regulierung der Nährstoffversorgung innerhalb der ECM und/oder zu einer Stimulierung der Aufbauprozesse der ECM führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird. Erfindungsgemäß bevorzugt zu verwendende Polysaccharide sind solche, die mindestens einen Desoxyzucker-Baustein, insbesondere Fucose und/oder Rhamnose, enthalten, besonders bevorzugt ausgewählt aus Substanzen mit den INCl-Bezeichnungen Biosaccharide Gum-1, Biosaccharide Gum-2, Biosaccharide Gum-3 und Biosaccharide Gum-4, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3. Weitere erfindungsgemäß bevorzugt zu verwendende Polysaccharide sind ausgewählt aus den Glycosaminoglycanen, besonders bevorzugt Hyaluronsäure und deren Salze, Dextran, Dextransulfat, Chondroitin-4-sulfat und Chondroitin-6-sulfat. Weitere erfindungsgemäß bevorzugt zu verwendende Polysaccharide sind ausgewählt aus den Glucanen. Bei Glucanen oder Polyglucosanen handelt es sich um eine Klasse von Homopolysacchariden, deren Monomerbaustein ausschließlich Glucose ist. Beispiele für Glucane sind Cellulose, Amylose, Glycogen, Lichenin, Laminarin aus Algen, Pachyman aus Baumpilzen und Hefeglucane mit β-1,3-Bindung; Nigeran, ein aus Pilzen isoliertes Mycodextran (α-1,3-Glucan, α-1,4-Glucan), Pustulan (β-1,6-Glucan), Dextran, Curdlan (β-1,3-D-Glucan), Pullulan und Schizophyllan. Weitere erfindungsgemäß bevorzugt zu verwendende Polysaccharide, die in Gegenwart der erfindungsgemäßen Zusammensetzung, eine unerwartete Wirkungssteigerung zeigen, sind ausgewählt aus chemisch modifizierten Cellulose-Derivaten, vorzugsweise Hydroxyalkylcellulosen, wie Hydroxyethylcellulose, Hydroxypropylcellulose, Hydroxypropylmethylcellulose, Methylcellulose, Hydroxymethylcellulose, Carboxymethylcellulose, weiterhin insbesondere aus Stärken sowie Stärkeabbauprodukten wie Amylose und Amylopektin, chemisch und/oder thermisch modifizierte Stärken, z. B. Hydroxypropylstärkephosphat, Dihydroxypropyldistärkephosphat, Aluminiumstärkeoctenylsuccinat, Natriumstärkeoctenylsuccinat oder Calciumstärkeoctenylsuccinat, weiterhin Chitosan und dessen Salzen und anderen Derivaten, weiterhin aus Fucose- und Rhamnose-freien Polysacchariden, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

Erfindungsgemäß besonders bevorzugte Zusammensetzungen enthalten mindestens ein Polysaccharid in einer Gesamtmenge von 0,001 - 10 Gew.-%, bevorzugt 0,01 - 5 Gew.-%, besonders bevorzugt 0,1 - 3 Gew.-% und außerordentlich bevorzugt 0,5 - 1 Gew.-%, jeweils bezogen auf den Gehalt an Aktivsubstanz Polysaccharid in der gesamten Zusammensetzung, enthalten.
Erfindungsgemäß besonders bevorzugte Zusammensetzungen enthalten Hyaluronsäure(salz) und eine antioxidative Substanz, ausgewählt aus TPP-VitC oder TPP-VitE oder TPP-MitQ^{®}. Weitere erfindungsgemäß besonders bevorzugte Zusammensetzungen enthalten beta-Glucan und/oder Natriumstärkeoctenylsuccinat und eine antioxidative Substanz, ausgewählt aus TPP-VitC oder TPP-VitE oder TPP-MitQ^{®}.

Weiterhin ist es erfindungsgemäß bevorzugt, die erfindungsgemäßen Zusammensetzungen mit Mischungen aus zwei oder mehreren der genannten Wirkstoffe, welche ausgewählt sind aus Aporphin-Alkaloiden, Purin und/oder Purinderivaten, natürlichen Betainverbindungen, alkyl- oder hydroxyalkylsubstituierten Harnstoffverbindungen, Monomeren, Oligomeren und Polymeren von Aminosäuren, N-C₂-C₂₄-Acylaminosäuren und/oder den Estern und/oder den physiologisch verträglichen Salzen dieser Substanzen, sowie Polysacchariden, einzusetzen.

Weitere erfindungsgemäß bevorzugte Zusammensetzungen enthalten mindestens einen weiteren Wirkstoff, ausgewählt aus Retinoiden, insbesondere Retinol und C₂-C₂₂-Fettsäureestern des Retinols, Oleanolsäure, Oleanol, Kreatin, Flavonoiden und Flavonoid-reichen Pflanzenextrakten, Isoflavonoiden und Isoflavonoid-reichen Pflanzenextrakten, Dihydroquercetin, Dillextrakt (Peucedanum Graveolens Extract), DNA- oder RNA-Oligonucleotiden, Vitaminen, Provitaminen und Vitaminvorstufen der Gruppen B und E und den Estern der vorgenannten Substanzen, α-Hydroxycarbonsäuren, α-Ketocarbonsäuren, β-Hydroxycarbonsäuren und deren Ester-, Lacton- und/oder Salzform, Silymarin, Ectoin, selbstbräunenden, hautberuhigenden und sebumregulierenden Wirkstoffen sowie Mischungen der vorgenannten Substanzen.

Zu den Isoflavonoiden zählen erfindungsgemäß die Isoflavone und die Isoflavon-Glycoside. Unter Isoflavonen sind erfindungsgemäß Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Besonders bevorzugte Isoflavone sind Daidzein, Genistein, Glycitein und Formononetin. Erfindungsgemäß besonders bevorzugte Isoflavon-Glycoside sind Daidzin und Genistin.

Zur Vitamin B-Gruppe oder zu dem Vitamin B-Komplex gehören unter anderem Vitamin B₁, Vitamin B₂, Vitamin B₃, insbesondere Nicotinsäure und Nicotinsäureamid (Niacinamid), Vitamin B₅, insbesondere Pantothensäure und Panthenol sowie Ester, Ether und kationische Derivate des Panthenols, außerdem Dihydro-3-hydroxy-4,4-dimethyl-2(3H)-furanon mit dem Trivialnamen Pantolacton; Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht, Vitamin B₇ (Biotin), Folsäure (Vitamin B₉, Vitamin B_{c}) und Orotsäure (Vitamin B₁₃). Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan.

Erfindungsgemäß besonders bevorzugte kosmetische oder dermatologische Zusammensetzungen enthalten mindestens eine der folgenden Wirkstoffkombinationen: Folsäure und eine antioxidative Substanz, ausgewählt aus TPP-VitC oder TPP-VitE oder TPP-MitQ^{®}, Folsäure und N-Palmitoyl-Lys-Thr-Thr-Lys-Ser und eine antioxidative Substanz, ausgewählt aus TPP-VitC oder TPP-VitE oder TPP-MitQ^{®}, Carnitin und Folsäure und eine antioxidative Substanz ausgewählt aus TPP-VitC oder TPP-VitE oder TPP-MitQ^{®}.

Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und Calcium-Salze. Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Weitere erfindungsgemäß besonders bevorzugte kosmetische oder dermatologische Zusammensetzungen enthalten Coenzym Q 10 und mindestens eine antioxidative Substanz, ausgewählt aus TPP-VitC oder TPP-VitE oder TPP-MitQ^{®}. Dies hat den besonderen Vorteil, dass die auf die Mitochondrien gerichtete antioxidative Substanz in der Zusammensetzung nicht durch Luftsauerstoff bereits oxidiert und damit unbrauchbar wird.

Weitere erfindungsgemäß besonders bevorzugte kosmetische oder dermatologische Zusammensetzungen enthalten Ectoin und mindestens eine antioxidative Substanz, ausgewählt aus TPP-VitC oder TPP-VitE oder TPP-MitQ^{®}.

Erfindungsgemäß bevorzugte hautberuhigende Wirkstoffe sind ausgewählt aus Allantoin, α-Bisabolol, α-Liponsäure, Extrakten aus Centella asiatica, Glycyrrethinsäure, dem Dipeptid Tyr-Arg, dessen Estern und dessen N-Acyl-Derivaten, insbesondere N-Acetyl-Tyr-Arg-hexyldecylester, sowie beliebigen Mischungen dieser Substanzen. Die hautberuhigenden Wirkstoffe sind bevorzugt in Mengen von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten. Erfindungsgemäß bevorzugte sebumregulierende Wirkstoffe sind ausgewählt aus Azelainsäure, Sebacinsäure, 10-Hydroxydecansäure, 1,10-Decandiol, Potassium Azeloyl Diglycinate, Extrakten aus Spiraea Ulmaria, weiterhin aus wasser- und öllöslichen Extrakten aus Hamamelis, Klettenwurzel und Brennessel, Zimtbaumextract und Chrysanthemenextrakt.

Erfindungsgemäß ist es bevorzugt, dass die Zusammensetzung eine topisch anzuwendende Zusammensetzung ist. So kann die Zusammensetzung auf geeignete Weise und ohne störende Nebenwirkungen auf andere Organe dort eingesetzt werden, wo sie ihre besonders geeignete Wirkung entfaltet, auf der Haut. Gemäß einer bevorzugten Ausführungsform der Erfindung ist die erfindungsgemäße Zusammensetzung, enthaltend eine antioxidative Substanz, die einen antioxidativen Rest trägt, der über eine Linker-Gruppe mit einer lipophilen, kationisch geladenen Gruppe verknüpft ist, sowie 0,0001 bis 99 Gew.-%, bezogen auf das Gesamtgewicht der Zusammensetzung, mindestens eines Tensids, in einer Form konfektioniert, die für eine Verabreichung auf topischem Wege geeignet ist.

Erfindungsgemäß bevorzugte Zusammensetzungen enthalten mindestens einen konditionierenden Wirkstoff, ausgewählt aus Fettstoffen, insbesondere pflanzlichen Ölen, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆-₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder HydroxyGruppen enthalten, bevorzugt Substanzen mit den INCI-Bezeichnungen Dimethiconol, Amodimethicone oder Trimethylsilylamodimethicone. Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte Zusammensetzung.

Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein. Gemäß einer weiteren bevorzugten Ausführungsform der Erfindung ist in der erfindungsgemäßen Zusammensetzung weiterhin mindestens ein Hilfs- und/oder Zusatzstoff enthalten, ausgewählt aus Verdickungsmitteln und anorganischen oder organischen UV-Filtersubstanzen. Weitere bevorzugte Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Ein weiterer Gegenstand der vorliegenden Erfindung ist die nicht-therapeutische, kosmetische Verwendung einer antioxidativen Substanz, die einen antioxidativen Rest trägt, der über eine Linker-Gruppe mit einer lipophilen, kationisch geladenen Gruppe verknüpft ist, zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung.

Ein weiterer Gegenstand der vorliegenden Erfindung ist die Verwendung mindestens einer antioxidativen Substanz, die einen antioxidativen Rest trägt, der über eine Linker-Gruppe mit einer lipophilen, kationisch geladenen Gruppe verknüpft ist, zur Herstellung einer Zusammensetzung zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung.

Für die erfindungsgemäßen Verwendungen gilt hinsichtlich bevorzugter Ausführungsformen mutatis mutandis das zu den erfindungsgemäßen Zusammensetzungen Gesagte.

Insbesondere werden die vorstehend beschriebenen erfindungsgemäßen Zusammensetzungen zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung bzw. zur Herstellung einer Zusammensetzung zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung verwendet.

Die nachfolgenden Formulierungsbeispiele sollen den Gegenstand der Erfindung erläutern, ohne ihn hierauf zu beschränken.

Alle Mengenangaben sind in Gew.-%, bezogen auf die gesamte Zusammensetzung.
1. Beispielserie: Wasser-in-Öl-Emulsionen

| | **1** | **2** | **3** |
|---|---|---|---|
| Dow Corning 245 Fluid | 25 | 25 | 25 |
| Abil EM 90 | 2 | 2 | 2 |
| Belsil DM 100 | 2,5 | 2,5 | 2,5 |
| Hydrogenated Castor Oil | 2 | 2,5 | 2 |
| Glycerin | 5 | 5 | 5 |
| NaCl | 2 | 2,1 | 2 |
| Symdiol 68 | 0,5 | 0,5 | 0,5 |
| Phenoxyethanol | 0,4 | 0,4 | 0,4 |
| D-Panthenol | 0,45 | 0,45 | 0,45 |
| D,I-alpha-bisabolol | 0,1 | 0,1 | 0,1 |
| TPP-VitE | 0,001 | 0,01 | 0,1 |
| Parfum | 0,3 | 0,3 | 0,3 |
| Wasser | Ad100 | Ad100 | Ad100 |

2. Beispielserie: Öl-in-Wasser-Emulsionen

| | **1** | **2** | **3** |
|---|---|---|---|
| Montanov 202 | 5,0 | 5,0 | 5,0 |
| Cutina MD | 2,0 | 2,0 | 2,0 |
| Cetearyl Alcohol | 1,0 | 1,0 | 1,0 |
| Cetiol B | 9,0 | 9,0 | 9,0 |
| Controx KS | 0,05 | 0,05 | 0,05 |
| Dow Corning 245 | - | 7,0 | 3,0 |
| Dow Corning 9040 | 1,0 | 1,0 | - |
| Parfum | 0,3 | 0,3 | 0,3 |
| Fucogel 1000 | - | 2,0 | - |
| Herbasol Destillat Salbei | - | - | 1,0 |
| Herbasol Destillat Hamamelis | - | - | 1,0 |
| D-Panthenol | - | 0,7 | 0,7 |
| Herbasol Destilat weisser Tee | - | 0,5 | - |
| Hydroglycolic Extract of Ginseng | - | 0,45 | - |
| DSH-C N | 5,0 | 5,0 | - |
| Keltrol SF | - | 0,2 | 0,2 |
| Aristoflex AVC | - | - | 0,5 |
| Hexandiol-1,6 | 6,0 | 6,0 | - |
| Glycerin | 3,0 | 5,0 | 10,0 |
| Dow Corning 200 Fluid | 7,0 | 7,0 | - |
| Dow Corning 245 | - | 5,0 | 3,0 |
| Dow Corning 9040 | 1,0 | - | 1,0 |
| Propylenglycol | 2,0 | 2,0 | - |
| Tego Carbomer | 0,3 | 0,3 | 0,3 |
| Dry Flo Plus | 1,0 | 1,0 | 1,0 |
| TPP-VitE | 0,001 | 0,01 | 0,1 |
| Wasser | ad 100 | ad 100 | ad 100 |

3. Hautcremes
Die Zusammensetzungen 1 - 4 sind bevorzugte Ausführungsformen von schützenden Tagescremes.

| | **1** | **2** | **3** | **4** |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-Öl M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| TPP-MitoQ^{®} | 0,01 | 0,01 | 0,01 | 0,001 |
| Caomint | 1,00 | 0,50 | 2,00 | - |
| Calmosensine | 1,00 | 2,00 | - | 2,00 |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Kombuchka | 3,00 | - | - | 3,00 |
| Glistin | - | 2,00 | - | - |
| Natrulon RC 50 DG | - | - | 1,00 | - |
| Phytokine | 2,00 | 1,50 | 2,00 | 2,00 |
| Ridulisse C | 0,50 | 0,50 | 0,50 | 0,50 |
| Liftiline | 2,00 | 3,00 | 2,00 | 3,00 |
| Matrixyl 3000 | - | 2,00 | - | - |
| Perfume | 0,10 | 0,10 | 0,10 | 0,10 |
| Aqua | ad 100 | ad 100 | ad 100 | ad 100 |

4. Hautcremes:
Die Zusammensetzungen 1 - 3 sind weitere bevorzugte Ausführungsformen von schützenden Tagescremes für besonders anspruchsvolle Haut mit einem Gehalt an Antiageing-Wirkstoffen.

| | 1 | 2 | 3 |
|---|---|---|---|
| Cetearyl Isononanoate | 4,00 | 4,00 | 4,00 |
| Mineral oil | 6,00 | 6,00 | 6,00 |
| Cutina CBS | 2,00 | 2,00 | 2,00 |
| Palmitic Acid / Stearic Acid | 1,50 | 1,50 | 1,50 |
| Eumulgin B3 | 1,00 | 1,00 | 1,00 |
| Baysilone-Öl M 350 | 1,00 | 1,00 | 1,00 |
| Tocopheryl Acetate | 0,50 | 0,50 | 0,50 |
| Propylparaben | 0,30 | 0,30 | 0,30 |
| Pemulen TR 1 | 0,27 | 0,27 | 0,27 |
| Glycerin | 5,00 | 3,00 | 3,00 |
| Milchsäure 80% | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Phytokine | 2,00 | 1,50 | 2,00 |
| Ridulisse C | 0,50 | 0,50 | 0,50 |
| Liftiline | 2,00 | 3,00 | 2,00 |
| Sepigel 305 | 0,50 | 0,50 | 0,50 |
| TPP-VitC | 0,1 | 0,025 | 0,001 |
| Taurin | 1,00 | 0,50 | 2,00 |
| Calmosensine | 1,00 | 2,00 | - |
| Keltrol SF | 0,20 | 0,20 | 0,20 |
| N,N-Bis-(2-hydroxyethyl)harnstoff | 4,30 | 2,50 | 8,60 |
| Perfume | 0,30 | 0,30 | 0,30 |
| Natrium Ascorbylphosphat | 1,00 | - | 1,00 |
| Keratec Pep | - | 2,00 | - |
| Kreatin | - | - | 1,00 |
| Retinol | 0,10 | - | - |
| Deliner | - | 2,00 | - |
| Wasser | ad 100 | ad 100 | ad 100 |

5. Tagescremes:
Die Zusammensetzungen 1 - 5 sind bevorzugte Ausführungsformen von schützenden Tagescremes für besonders anspruchsvolle Haut mit einem Gehalt an Antiageing-Wirkstoffen.

| | 1 | 2 | 3 | 4 | 5 |
|---|---|---|---|---|---|
| Lipoid S 75-3 | 0,50 | 0,50 | 0,50 | 0,50 | 0,50 |
| Isopropylstearate | 4,00 | 4,00 | 4,00 | 4,00 | 4,00 |
| Dibutyl Adipate | 2,00 | 2,00 | 2,00 | 2,00 | 2,00 |
| Tocopheryl Acetate | 0,50 | 0,50 | 0,50 | 0,50 | 0,50 |
| Cutina MD | 1,00 | 1,00 | 1,00 | 1,00 | 1,00 |
| Behenyl Alcohol | 2,00 | 2,00 | 2,00 | 2,00 | 2,00 |
| Baysilone-Öl M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerol | 3,00 | 4,50 | 3,00 | 3,00 | 3,00 |
| Hexandiol | 6,00 | 3,00 | - | - | 4,00 |
| Methylparaben | 0,20 | 0,20 | 0,20 | 0,20 | 0,20 |
| Tego Carbomer | 0,30 | 0,30 | 0,30 | 0,30 | 0,30 |
| Algae Extract | 1,00 | - | - | - | - |
| Photosomes | 0,20 | 0,20 | 0,20 | 0,20 | 0,20 |
| Dimethylmethoxy Chromanol | 0,01 | 0,01 | 0,01 | 0,01 | 0,01 |
| Propylenglycol | 5,00 | 5,00 | 5,00 | 5,00 | 5,00 |
| Phytokine | 2,00 | 1,50 | 2,00 | 2,00 | 1,50 |
| Ridulisse C | 0,50 | 0,50 | 0,50 | 0,50 | 0,50 |
| Liftiline | 2,00 | 3,00 | 2,00 | 2,00 | 3,00 |
| TPP-VitE | 0,01 | 0,10 | 0,050 | 0,01 | 0,0020 |
| Calmosensine | 1,00 | 2,00 | - | 1,00 | 1,00 |
| Kombuchka | 3,00 | 3,00 | - | - | 2,00 |
| Natrium Ascorbylphosphat | - | - | 1,00 | 1,00 | 1,00 |
| Natrulon RC50DG | - | - | - | - | 1,00 |
| Deliner | - | - | 2,00 | 1,00 | - |
| Matrixyl 3000 | 1,00 | 1,00 | - | - | 1,00 |
| Simulgel NS | 1,50 | 1,50 | 1,50 | 1,50 | 1,50 |
| Sodium Benzoate | - | 0,20 | 0,50 | - | - |
| Phenoxyethanol | 0,50 | - | - | - | - |
| Symdiol 68 | 0,30 | 0,50 | 0,30 | - | - |
| TiO₂ | 0,50 | 0,50 | - | - | - |
| Hydrovance | 2,50 | 4,30 | 8,60 | 8,60 | 8,60 |
| Silymarin Phytosome | 0,30 | - | - | - | 0,50 |
| Ectoin | 0,50 | - | 0,50 | 0,50 | 0,50 |
| Vitamin B6 | - | - | 0,01 | 0,01 | 0,01 |
| Perfume | 0,35 | 0,35 | 0,35 | 0,35 | 0,35 |
| Aqua | ad 100 | ad 100 | ad 100 | ad 100 | ad 100 |

6. Pflegecremes
Die Zusammensetzungen 1 - 6 sind bevorzugte Ausführungsformen von schützenden Pflegecremes mit gel-ähnlicher Konsistenz.

| | 1 | 2 | 3 | 4 | 5 | 6 |
|---|---|---|---|---|---|---|
| 2-Ethylhexylpalmitat | 2,00 | 2,00 | 2,00 | 2,00 | 2,00 | 2,00 |
| Dow Corning 1403 Fluid | 5,00 | 5,00 | 5,00 | 5,00 | 5,00 | 5,00 |
| Synperonic PE L 64 | 2,00 | 2,00 | 2,00 | 2,00 | 2,00 | 2,00 |
| Dow Corning 9040 | 5,00 | 5,00 | 5,00 | 5,00 | 5,00 | 5,00 |
| Dipropylenglykol | 5,00 | 5,00 | 5,00 | 5,00 | 5,00 | 5,00 |
| Tego Carbomer 140 | 0,70 | 0,70 | 0,70 | 0,70 | 0,70 | 0,70 |
| Parfum | 0,35 | 0,30 | 0,35 | 0,35 | 0,30 | 0,30 |
| Phenoxyethanol, rein | 0,40 | 0,40 | 0,40 | 0,40 | 0,40 | 0,40 |
| Natrulon RC-50DG | - | 0,75 | 0,75 | 0,75 | 0,75 | 0,75 |
| Menthyl Lactate | 0,50 | 0,50 | 0,50 | 0,50 | 0,50 | 0,50 |
| Ethanol 96 % DEP vergällt | 5,00 | 5,00 | 5,00 | 5,00 | 5,00 | 5,00 |
| Coffein wasserfrei | 0,50 | - | 0,50 | 0,50 | 0,50 | 0,50 |
| Ridulisse C | - | - | - | 0,50 | - | - |
| TPP-VitE | 0,0050 | 0,0050 | 0,0050 | 0,01 | 0,01 | 0,01 |
| Simulgel NS | 0,75 | 0,75 | 0,75 | 0,75 | 0,75 | 0,75 |
| Matrixyl | - | - | - | - | 2,00 | - |
| Matrixyl 3000 | - | - | - | - | - | 2,00 |
| Natriumhydroxid Perlen | 0,081 | 0,081 | 0,081 | 0,081 | 0,081 | 0,081 |
| Wasser, vollentsalzt | ad 100,00 | ad 100,00 | ad 100,00 | ad 100,00 | ad 100,00 | ad 100,00 |

7. Die Zusammensetzungen 1 - 5 sind bevorzugte Ausführungsformen von schützenden Tagescremes mit höherem Lichtschutzfilter (SPF ca. 10 - 15) für besonders anspruchsvolle Haut mit einem Gehalt an Antiageing-Wirkstoffen.

| | 1 | 2 | 3 | 4 | 5 |
|---|---|---|---|---|---|
| Montanov 68 | 5,00 | 5,00 | 5,00 | 5,00 | 5,00 |
| Myritol 318 | 5,00 | 5,00 | 5,00 | 5,00 | 5,00 |
| Cetiol SB 45 | 0,50 | 0,50 | 0,50 | 0,50 | 0,50 |
| Novata AB | 2,00 | 2,00 | 2,00 | 2,00 | 2,00 |
| Cetearylalkohol | 1,00 | 1,00 | 1,00 | 1,00 | 1,00 |
| Baysilon M 350 | 0,50 | 0,50 | 0,50 | 0,50 | 0,50 |
| Tocopherylacetat | 0,50 | 0,50 | 0,50 | 0,50 | 0,50 |
| Controx KS | 0,25 | 0,25 | 0,25 | 0,25 | 0,25 |
| Parsol SLX | - | 4,00 | 4,00 | - | - |
| Parsol HS | - | 2,00 | 2,00 | 2,00 | - |
| Neo Heliopan AP | - | - | - | 1,00 | - |
| Parsol 340 | - | - | - | 5,00 | - |
| Uvinul MBC 95 | 2,00 | - | - | - | - |
| Parsol 1789 | 1,00 | 1,80 | 1,80 | - | - |
| Propylparaben | 0,20 | 0,20 | 0,20 | 0,20 | 0,20 |
| Tego Carbomer 140 | 0,50 | 0,50 | 0,50 | 0,50 | 0,50 |
| 1,6-Hexandiol | 6,00 | 6,00 | 3,00 | 6,00 | - |
| Talkum | 0,50 | 0,50 | 0,50 | 0,50 | 0,50 |
| Methylparaben | 0,20 | 0,20 | 0,20 | 0,20 | 0,20 |
| Glycerin | 4,50 | 4,50 | 3,00 | 4,50 | 3,00 |
| Dry Flo Plus | 1,00 | 1,00 | 1,00 | 1,00 | 1,00 |
| Natipide 2 PG | 1,00 | 1,00 | 1,00 | 1,00 | 1,00 |
| Phenoxyethanol | 0,40 | 0,40 | 0,40 | 0,40 | 0,40 |
| TPP-VitC | 0,01 | 0,0050 | 0,01 | 0,01 | 0,01 |
| Carnitin | - | 0,10 | 0,50 | - | - |
| Matrixyl 3000 | 1,00 | - | - | 4,00 | 2,00 |
| Ridulisse C | - | - | 3,00 | - | 0,50 |
| Parfum | 0,40 | 0,40 | 0,40 | 0,40 | 0,40 |
| Aqua | ad 100 | ad 100 | ad 100 | ad 100 | ad 100 |

8. Die Zusammensetzungen 1 - 3 sind bevorzugte Ausführungsformen von schützenden Gesichtswässern, die bevorzugt mit einem Pad, Bausch oder Tuch auf die Haut appliziert werden.

| | 1 | 2 | 3 |
|---|---|---|---|
| Dipropylenglycol | 10,00 | 10,00 | 10,00 |
| Chlorhexidindigluconat | 1,00 | 1,00 | 1,00 |
| Synperonic PE7 L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil Trideceth 9 / Propylene Glycol | / 0,50 | 0,50 | 0,50 |
| Caomint | 0,50 | 0,50 | 0,50 |
| TPP-VitC | 0,01 | 0,0025 | 0,0050 |
| Ridulisse C | 1,00 | - | - |
| Fucogel 1000 | - | - | 1,00 |
| Betain | - | 1,00 | - |
| Parfum | 0,20 | 0,20 | 0,20 |
| Aqua | ad 100 | ad 100 | ad 100 |

9. Die Zusammensetzungen 1 - 3 sind bevorzugte Ausführungsformen von schützenden Cremes auf der Basis einer reichhaltigen und dennoch nicht-fettenden Wasser-in-Silicon-Emulsion, die in die Haut einmassiert werden kann.

| | 1 | 2 | 3 |
|---|---|---|---|
| Belsil DM 100 | 2,50 | 2,50 | 2,50 |
| Dow Corning 245 Fluid | 25,00 | 25,00 | 25,00 |
| Abil EM 90 | 2,00 | 2,00 | 2,00 |
| Natriumchlorid | 2,00 | 2,00 | 2,00 |
| Symdiol 68 | 0,30 | 0,30 | 0,30 |
| Phenoxyethanol | 0,40 | 0,40 | 0,40 |
| TPP-VitC | 0,01 | 0,01 | 0,01 |
| Ridulisse C | - | 2,00 | 1,00 |
| Rhamnosoft | 2,00 | - | - |
| Hydrovance | - | 3,00 | - |
| Folsäure | - | - | 0,10 |
| Parfum | 0,30 | 0,30 | 0,30 |
| Aqua | ad 100 | ad 100 | ad 100 |

10. Tagescreme:
Die folgende Zusammensetzung ist eine weitere bevorzugte Ausführungsform einer schützenden erfindungsgemäßen Zusammensetzung.

| | |
|---|---|
| Lipoid S 75-3 | 1,000000 |
| Isopropylstearat | 2,000000 |
| Cetiol B | 4,000000 |
| Vitamin E Acetat | 0,500000 |
| Cutina MD | 1,000000 |
| Cetearyl Alcohol | 2,500000 |
| NOVATA AB PH | 0,500000 |
| Behenylalkohol | 3,500000 |
| Silikonöl 350 cs | 1,500000 |
| Propylparaben | 0,200000 |
| Dow Corning 9040 | 2,000000 |
| Glycerin 86% | 8,000000 |
| Hexandiol-1,6 | 6,000000 |
| Methylparaben | 0,200000 |
| Talkum | 3,000000 |
| Tego Carbomer 140 | 0,400000 |
| Hydroglycolic Extract of Caviar | 1,000000 |
| TPP-VitE | 0,00500000 |
| Ederline L | 1,000000 |
| DSH-C N | 3,000000 |
| Photosomes | 0,200000 |
| Lipochroman-6 | 0,010000 |
| Propandiol-1,2 | 4,000000 |
| Matrixyl 3000 | 3,000000 |
| Phenoxyethanol, rein | 0,400000 |
| Parfum | 0,200000 |
| RONASPHERE LDP | 1,000000 |
| FD&C Yellow No. 6 | 0,000700 |
| Simulgel NS | 1,500000 |
| Natriumhydroxid | 0,040000 |
| Wasser, vollentsalzt | ad 100,00000 |

11. Reinigungszubereitungen:

| | **1** | **2** | **3** |
|---|---|---|---|
| Dipropylenglycol | 10,00 | 10,00 | 10,00 |
| Chlorhexidindigluconat | 1,00 | 1,00 | 1,00 |
| Synperonic PE7L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylen Glycol | 0,50 | 0,50 | 0,50 |
| TPP-VitC | 0,005 | 0,01 | 0,1 |
| Parfume | 0,20 | 0,20 | 0,20 |
| Aqua | ad. 100 | ad. 100 | ad. 100 |

12. Beispiele für Zusammensetzungen zum Tränken von Tüchern

| | Bestandteile | 1 | 2 | 3 | 4 |
|---|---|---|---|---|---|
| Phase 1 | PEG-40 Hydrogenated Castor Oil | 0,1 | 0,1 | 0,1 | - |
| | Trideceth-9 | 0,1 | 0,1 | 0,1 | - |
| | Parfum | 0,3 | 0,1 | 0,1 | 0,1 |
| Phase 2 | Hexyllaurat | 2,0 | - | - | - |
| | Dicaprylylcarbonat | - | - | 2,0 | - |
| | Dipropylenglycol | - | 2,0 | - | - |
| Phase 3 | Allantoin | 0,5 | - | - | - |
| | Citronensäure (Monohydrat) | 1,5 | 1,5 | 1,5 | 1,5 |
| | Trinatriumcitrat (Dihydrat) | 2,0 | 2,0 | 2,0 | 2,0 |
| | Decylglucosid | - | - | - | 2,0 |
| | Pemulen TR 1 | - | - | - | 0,2 |
| | TPP-VitE | 0,03 | 0,02 | 0,003 | 0,01 |
| | Ridulisse C | 1,00 | 0,50 | 1,00 | - |
| | Carnitin | 0,20 | - | - | 0,50 |
| | Wasser | ad 100 | ad 100 | ad 100 | ad 100 |

Mit diesen Tränkzusammensetzungen wurden verschiedene Viskose-Vliesstoffe ausgerüstet. Es wurden gelochte, ungelochte, genoppte, einlagige, zweilagige und dreilagige Vliese ausgerüstet. Die Tücher wurden sowohl als feuchte Tücher als auch als trockene Tücher (das heißt, nach dem Ausrüsten wurden die Tücher bis auf einen Restwassergehalt kleiner 10 Gew.-%, bevorzugt kleiner 5 Gew.-%, getrocknet, um direkt vor Gebrauch mit einer kleinen Menge an Wasser wieder befeuchtet zu werden oder auf feuchter Haut angewendet zu werden) verwendet.

### Matrixpflaster mit schützendem Effekt

| | 1 | 2 | 3 | 4 | 5 |
|---|---|---|---|---|---|
| DURO-TAK^{®} 387-2516 | 76 | 76 | 76 | 76 | 76 |
| Aloe Vera Gel | 1 | - | 1 | - | - |
| Propylenglycolmonooleat | 5 | 5 | - | - | - |
| Tween^{®}-80 | - | - | 5 | 3 | 5 |
| Natriumcitrat | 0,1 | 0,1 | 0,1 | 0,1 | 0,1 |
| Controx^{®} KS | 0,05 | 0,05 | 0,05 | 0,05 | 0,05 |
| TPP-VitC | 0,03 | 0,03 | 0,03 | 0,01 | 0,03 |
| Ridulisse C | 1,00 | 0,50 | 1,00 | - | - |
| Carnitin | 0,20 | - | - | 0,50 | 0,30 |
| Matrixyl 3000 | 3,00 | - | - | - | 2,00 |
| Wasser | ad 100 | ad 100 | ad 100 | ad 100 | ad 100 |

Die nachfolgendenRezepturen sind bevorzugte Beispiele für erfindungsgemäße schützende Körpercremes:

| | | |
|---|---|---|
| Paraffinum Liquidum | 20,00 | 20,00 |
| Arlacel 165 | 4,00 | 4,00 |
| Octyldodecanol | 1,00 | 1,00 |
| Cetiol SN | 1,50 | 1,50 |
| Cremophor A 6 | 2,00 | 2,00 |
| Behenylalkohol | 1,60 | 1,60 |
| Silikonöl 350 cs | 3,00 | 3,00 |
| Brij 721 VP | 0,40 | 0,40 |
| Propylparaben | 0,20 | 0,20 |
| Controx KS | 0,25 | 0,25 |
| Propandiol-1,2 | 3,00 | 3,00 |
| Glycerin 86% pflanzlich | 5,00 | 5,00 |
| Polyethylenglykol MG 400 | 1,70 | 1,70 |
| Methylparaben | 0,20 | 0,20 |
| Phenoxyethanol, rein | 0,50 | 0,50 |
| Carbopol ETD 2020 | 0,50 | 0,50 |
| Natriumhydroxid | 0,03 | 0,03 |
| Citronensäure Monohydrat | 0,10 | 0,10 |
| Trinatriumcitrat Dihydrat | 0,10 | 0,10 |
| TPP-VitC | 0,03 | 0,03 |
| Sensiva SC 50 (Octoxyglycerin) | 0,50 | 0,50 |
| Carnitin | 0,20 | 0,20 |
| Ergothionein | - | 0,10 |
| Parfum | 0,30 | 0,30 |
| Simulgel NS | 1,00 | 1,00 |
| Wasser | ad 100,00 | ad 100,00 |

### Liste der verwendeten Rohstoffe

| Handelsname | INCI-Bezeichnung | Lieferant/Hersteller |
|---|---|---|
| Abil EM 90 | CETYL PEG/PPG-10/1 DIMETHICONE | Degussa |
| Arlacel 165 | Glyceryl Stearate, PEG-100 Stearate | Uniqema |
| Aristoflex AVC | Ammonium Acryloly-Dimethyltaurat/VP Copolymer, t-Butyl-Alcohol | Clariant |
| Baysilone-Öl M 350 | Dimethicone | GE Bayer Silicones |
| Belsil DM 100 | Dimethicone | Wacker |
| Brij 721 VP | STEARETH-21 | Uniqema |
| Calmosensine | Butylene Glycol, Water, Laureth-3, Hydroxy-ethylcellulose, Acetyl Dipeptide-1 Cetylester | Sederma |
| Caomint | Propylene Glycol, Water, Mentha Piperita (Pepermint) Leaf Extract, Theobroma Cacao (Cocoa) Extract | Solabia |
| Carbopol ETD 2020 | Acrylates/C10-30 Alkyl Acrylate Crosspolymer | Noveon |
| Cetiol^{®} B | Dibutyl Adipate | Cognis |
| Cetiol^{®} SB 45 | Butyrospermum parkii (Shea butter) | Cognis |
| Cetiol^{®} SN | Cetearyl Isononanoate | Cognis |
| Controx KS | Tocopherol, Hydrogenated Palm Glycerides Citrate | Cognis |
| Cremophor A 6 | CETEARETH-6, STEARYL ALCOHOL | BASF |
| Cutina CBS | Glyceryl Stearate / Cetearyl Alcohol / Cetyl alpitate / Cocoglycerides im Gewichtsverhältnis 7:1:1:1 | Cognis |
| Cutina MD | Glyceryl Stearate (Mischung aus Glycerylmono- und distearat) | Cognis |
| Deliner | Zea Mays (Corn) Kernel Extract, Butylene Glycol, Xanthan Gum | Coletica |
| Dow Corning 1403 Fluid | Cyclomethicone (86 - 88 Gew.-%), Dimethiconol (12 - 14 Gew.-%) | Dow Corning |
| Dow Corning 1501 Fluid | Cyclomethicone (85 - 86 Gew.-%), Dimethiconol (14-15 Gew.-%) | Dow Corning |
| Dow Corning 200 Fluid | Dimethicone | Dow Corning |
| Dow Corning 9040 | Cyclomethicone/ Dimethicone Crosspolymer (87-88%/12-13%) | Dow Corning |
| Dow Corning^{®} 245 Fluid | Cyclomethicone | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| Ederline L | Hexyldecanol, Pyrus Malus (Apple) Fruit Extract | Seporga |
| Eumulgin B 3 | Ceteareth-30 | Cognis |
| Fucogel 1000 | Biosaccharide Gum-1 | Solabia |
| Glistin | Water, L-Glutamylaminoethyl indole | Exsymol |
| Herbasol Destillat Salbei | Propylene Glycol, Aqua (Water), Saliva Officinalis (SAGE) Leaf Extract | Cosmetochem |
| Herbasol Destillat Hamamelis | Aqua (Water), Alcohol denat., Hamamelis Virginiana, Benzoic Acid, Polysorbate 20, Diethylphthalate | Cosmetochem |
| Herbasol Destillat weißer Tee | Propylene Glycol, Aqua (Water), Camellia Sinensis Leaf Extract | Cosmetochem |
| Hibiscin^{®} HP-LS-9198 | Water, Hibiscus esculentus Seed Extract, Phenoxyethanol | Laboratoires Sérobiologiques |
| Hydagen CMF | Chitosan Glycolate | Cognis |
| Hydrovance | Wasser, Bis-N,N'-(2-Hydroxyethyl)harnstoff, Harnstoff, Ammoniumlactat | National Starch |
| Keltrol SF | Xanthan Gum | Kelco |
| Keratec Pep | Water, Hydrolyzed Keratin | Croda |
| Kombuchka | Saccharomyces/Xylinum/Black Tea Ferment, Glycerin, Hydroxyethylcellulose | Sederma |
| Liftiline | Aqua, Hydrolyzed Wheat Protein (7 - 10 Gew.-% Aktivsubstanz) | Silab |
| Lipochroman 6 | DIMETHYLMETHOXY CHROMANOL | Lipotec |
| Lipoid S 75-3 | Aqua, Lecithine | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Montanov 202 | Arachidyl Alkohol, Behenyl Alcohol, Arachidyl Glucoside | Seppic |
| Myritol 318 | Caprylic/Capric Triglyceride | Cognis |
| Natipide 2 PG | Aqua, Propylene Glycol, Lecithin | Phospholipid GmbH |
| Natrulon RC 50 DG | WATER, CARNITINE, POLYGLYCERIN-10 | |
| Neo Heliopan AP | Disodium Phenyl Dibenzimidazole Tetrasulfonate | Symrise |
| Novata AB | Cocoglycerides | Cognis |
| Parsol 1789 | BUTYL METHOXYDIBENZOYLMETHANE | DSM |
| Parsol 340 | Octocrylene | DSM |
| Parsol HS | PHENYLBENZIMIDAZOLE SULFONIC ACID | DSM |
| Parsol SLX | Polysilicone-15 | DSM |
| Pemulen TR 1 | Acrylates/C10-30 Alkyl Acrylate Crosspolymer | Noveon |
| Photosomes | Aqua, Lecithine, Plancton Extract | AGI Dermatics |
| Phytokine | Aqua, Butylene Glycol, HYDROLYZED SOY PROTEIN, Methylparaben, Ethylparaben, Propylparaben (0,4 - 0,8 Gew.-% Aktivsubstanz) | Coletica |
| Rhamnosoft | Biosaccharide Gum-2 | Solabia |
| Ronasphere^{®} LDP | SILICA, CI 77891 (TITANIUM DIOXIDE), CI 77491 (IRON OXIDES) | Merck KGaA |
| Ridulisse C | Water, HYDROLYZED SOY PROTEIN (3 - 5 Gew.-% Aktivsubstanz) | Silab |
| Sensiva^{®} SC 50 | 2-Ethylhexylglycerinether | Schülke & Mayr |
| Sepigel^{®} 305 | Aqua (Water) Polyacrylamide, C₁₃-C₁₄ Isoparaffin, Laureth-7 (Aktivsubstanz Verdickerpolymer ca. 45 - 49 Gew.-%) | SEPPIC |
| Silymarin Phytosome | Silybum Marianum Extract and Phospholipids | Indena SpA |
| Simulgel NS | Aqua (Water)/Hydroxyethyl Acrylate / Sodium Acryloyldimethyl Taurate Copolymer / Squalane / Polysorbate 60 | Seppic |
| Symdiol 68 | 1,2-Octandiol, 1,2-Hexandiol | Symrise |
| Synperonic PE L 64 | Poloxamer-184 | Uniqema |
| Tego Carbomer 140 | Carbomer | Degussa |
| Tween^{®}-80 | Polysorbate-80 | |
| Uvinul MBC 95 | 4-METHYLBENZYLIDENE CAMPHOR | BASF |

## Patentansprüche

1. Zusammensetzung, enthaltend
a) mindestens eine antioxidative Substanz, die
i) einen antioxidativen Rest (A) trägt, welcher über eine Linker-Gruppe (L) mit einer lipophilen, kationisch geladenen Gruppe (K) verknüpft ist, und
ii) ein kosmetisch und/oder pharmazeutisch akzeptables Gegenion (Z) umfasst, sowie
b) 0,0001 bis 99 Gew.-%, bezogen auf das Gesamtgewicht der Zusammensetzung, mindestens eines Tensids.

2. Zusammensetzung nach Anspruch 1, **dadurch gekennzeichnet, dass** die lipophile, kationisch geladene Gruppe (K) ausgewählt ist aus substituierten oder unsubstituierten, bevorzugt unsubstituierten Triphenylphosphoniumkationen.

3. Zusammensetzung nach Anspruch 1 oder 2, **dadurch gekennzeichnet, dass** der antioxidative Rest (A) ausgewählt ist aus einem Butylhydroxytoluoylrest, Ascorbinsäureresten, insbesondere Ascorbinsäureethern, unsubstituierten oder substituierten 1,4-Hydrochinon- oder 1,4-Benzochinon-Resten, insbesondere ausgewählt aus 2,3-Dimethoxy-5-methyl-1,4-benzochinon-Resten, 1,4-Naphtochinol- und -chinon-Resten, insbesondere 2-Methyl-1,4-naphthochinol- und -chinon-Resten, 6,7-disubstituierten 2,2-Dialkylchroman- oder -chromen-Resten und unsubstituierten oder substituierten Chroman-6-ol-Resten, insbesondere 2,4,8-Trimethyl-Chroman-6-ol.

4. Zusammensetzung nach Anspruch 3, **dadurch gekennzeichnet, dass** der antioxidative Rest ausgewählt ist aus einem Ascorbinsäurerest, einem 3,4-Dihydro-2,4,8 Trimethyl-2H-1-benzopyran-6-ol-Rest oder einem 3,4-Dihydro-7-methoxy-2,2-dimethyl-2H-1-benzopyran-6-ol-Rest.

5. Zusammensetzung nach einem der Ansprüche 1 bis 4, **dadurch gekennzeichnet, dass** die Linker-Gruppe (L) durch eine unsubstituierte oder substituierte Alkyl-, Alkenyl- oder Alkinylkette mit 1 bis 17, bevorzugt 3 bis 15 Kohlenstoffatomen gebildet wird.

6. Zusammensetzung nach Anspruch 5, **dadurch gekennzeichnet, dass** die Linkergruppe eine unsubstituierte oder substituierte Alkylkette ist.

7. Zusammensetzung nach einem der Ansprüche 5 oder 6, **dadurch gekennzeichnet, dass** die substituierte Alkyl-, Alkenyl- oder Alkinylkette einen oder mehrere Substituenten umfasst, welche ausgewählt sind aus unsubstituierten oder substituierten Alkyl-, Alkenyl- oder Alkinylseitengruppen, sowie Hydroxyl-, Carboxy- und Amidgruppen.

8. Zusammensetzung nach einem der Ansprüche 1 bis 7, **dadurch gekennzeichnet, dass** das kosmetisch und/oder pharmazeutisch akzeptable Gegenion (Z) ausgewählt ist aus Alkylsulfonaten, insbesondere Methansulfonat, Ethansulfonat, Arylsulfonaten, insbesondere Benzolsulfonat, Methylbenzyolsulfonat und 2-Naphthalinsulfonat, Nitrat und Halogenen, insbesondere Bromid.

9. Zusammensetzung nach einem der Ansprüche 1 bis 8, **dadurch gekennzeichnet, dass** die antioxidative Substanz in einer Konzentration von 0,00001 bis 10 Gew.-%, bevorzugt zu 0,0001 bis 1 Gew.-%, bezogen auf das Gesamtgewicht der Zusammensetzung, enthalten ist.

10. Zusammensetzung nach einem der Ansprüche 1 bis 9, **dadurch gekennzeichnet, dass** mindestens ein Tensid, ausgewählt aus der Gruppe der nichtionischen Tenside, enthalten ist.

11. Zusammensetzung nach Anspruch 10, **dadurch gekennzeichnet, dass** das nichtionische Tensid ausgewählt ist aus
• Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
• C_{12/18}-Fettsäuremono- und -diestern von Anlagerungsprodukten von 1 bis 30 Mol Ethylenoxid an Glycerin;
• Glycerinmono- und -diestern und Sorbitanmono- und -diestern von gesättigten und ungesättigten Fettsäuren mit 6 bis 22 Kohlenstoffatomen und deren Ethylenoxidanlagerungsprodukten;
• Alkylmono- und -oligoglycosiden mit 8 bis 22 Kohlenstoffatomen im Alkylrest und deren ethoxylierten Analoga;
• Anlagerungsprodukten von 15 bis 60 Mol Ethylenoxid an Ricinusöl und/oder gehärtetes Ricinusöl;
• Polyolestern;
• Anlagerungsprodukten von 2 bis 15 Mol Ethylenoxid an Ricinusöl und/oder gehärtetes Ricinusöl;
• Partialestern auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipenta-erythrit, Zuckeralkoholen, Alkylglucosiden sowie Polyglucosiden;
• Trialkylphosphaten sowie Mono-, Di- und/oder Tri-PEG-Alkylphosphaten;
• Wollwachsalkoholen;
• Polysiloxan-Polyalkyl-Polyether-Copolymeren bzw. entsprechenden Derivaten;
• Mischestern aus Pentaerythrit, Fettsäuren, Citronensäure und Fettalkohol und/oder Misch-estern von Fettsäuren mit 6 bis 22 Kohlenstoffatomen, Methylglucose und Polyolen sowie
• Polyalkylenglycolen.

12. Zusammensetzung nach einem der Ansprüche 1 bis 11, **dadurch gekennzeichnet, dass** das mindestens eine Tensid zu 0,1 - 30 Gew.%, weiter bevorzugt 0,5 - 15 Gew.%, weiter bevorzugt 1,0 bis 10 Gew.-%, weiter bevorzugt 1,5 - 5 Gew.-%, jeweils bezogen auf das Gesamtgewicht der Zusammensetzung, enthalten ist.

13. Zusammensetzung nach einem der Ansprüche 1 bis 12, **dadurch gekennzeichnet, dass** die Zusammensetzung eine topisch anzuwendende Zusammensetzung ist.

14. Nicht-therapeutische, kosmetische Verwendung einer antioxidativen Substanz, die einen antioxidativen Rest trägt, welcher über eine Linker-Gruppe mit einer lipophilen, kationisch geladenen Gruppe verknüpft ist, zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung.

15. Verwendung mindestens einer antioxidativen Substanz, die einen antioxidativen Rest trägt, welcher über eine Linker-Gruppe mit einer lipophilen, kationisch geladenen Gruppe verknüpft ist, zur Herstellung einer Zusammensetzung zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung.


SEQUENCE LISTING

<110>  Henkel AG & Co. KGaA

<120>  Antioxidative Zusammensetzungen

<130>  H 08154 EP

<150>  DE 102008061340.1
<151>  2008-12-11

<160>  22

<170>  PatentIn version 3.3

<210>  1
<211>  4
<212>  PRT
<213>  Artificial

<220>
<223>  Artificial

<400>  1

Gly Gln Pro Arg
1


<210>  2
<211>  4
<212>  PRT
<213>  Artificial

<220>
<223>  N-Palmitoyl

<400>  2

Gly Gln Pro Arg
1


<210>  3
<211>  4
<212>  PRT
<213>  Artificial

<220>
<223>  Artificial

<400>  3

Ala Gln Thr Arg
1

<210>  4
<211>  4
<212>  PRT
<213>  Artificial

<220>
<223>  tetrapeptide


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Cirullin, Canavanin.

<220>
<221>  misc_feature
<222>  (4)..(4)
<223>  Xaa chosen from Pro or Arg and amino acids that can replace Pro
       or Arg, Arg replacing amino acids are preferably Pro, Lys His,
       Desmosin or Isodesmosin.

<400>  4

Xaa Xaa Arg Xaa
1


<210>  5
<211>  4
<212>  PRT
<213>  Artificial

<220>
<223>  Artificial Sequence


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Cirullin, Canavanin.

<220>
<221>  misc_feature

<222>  (3)..(3)
<223>  Xaa chosen from Pro or Arg and amino acids that can replace Pro
       or Arg, Arg replacing amino acids are preferably Pro, Lys His,
       Desmosin or Isodesmosin.

<400>  5

Xaa Xaa Xaa Pro
1


<210>  6
<211>  4
<212>  PRT
<213>  Artificial

<220>
<223>  Artificial Sequence


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Cirullin, Canavanin.

<220>
<221>  misc_feature
<222>  (4)..(4)
<223>  Xaa chosen from Pro or Arg and amino acids that can replace Pro
       or Arg, Arg replacing amino acids are preferably Pro, Lys His,
       Desmosin or Isodesmosin.

<400>  6

Xaa Xaa Pro Xaa
1


<210>  7
<211>  4
<212>  PRT
<213>  Artificial

<220>
<223>  Artificial Sequence


<220>
<221>  misc_feature
<222>  (1)..(1)

<223>  Xaa chosen from Gly and amino acids that can replace Gly,
      preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
      preferably Asn, Lys, Orn, 5-Hydroxyprolin, Cirullin, Canavanin.

<400>  7

Xaa Xaa Pro Arg
1


<210>  8
<211>  4
<212>  PRT
<213>  Artificial

<220>
<223>  Artificial Sequence


<220>
<221>  misc_feature
<222>  (1)..(1)
<223>  Xaa chosen from Gly and amino acids that can replace Gly,
      preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
      preferably Asn, Lys, Orn, 5-Hydroxyprolin, Cirullin, Canavanin.

<220>
<221>  misc_feature
<222>  (3)..(3)
<223>  Xaa chosen from Pro or Arg and amino acids that can replace Pro
      or Arg, Arg replacing amino acids are preferably Pro, Lys His,
      Desmosin or Isodesmosin.

<400>  8

Xaa Xaa Xaa Arg
1


<210>  9
<211>  4
<212>  PRT
<213>  Artificial

<220>
<223>  Artificial Sequence

<400>  9

Val Val Arg Pro
1

<210>  10
<211>  5
<212>  PRT
<213>  Artificial

<220>
<223>  preferably with N-Acyl or ester-bond

<400>  10

Lys Thr Thr Lys Ser
1               5

<210>  11
<211>  5
<212>  PRT
<213>  Artificial

<220>
<223>  Artificial Sequence

<220>
<221>  MOD_RES
<222>  (1)..(1)
<223>  N-Palmitoyl-Lys

<400>  11

Lys Thr Thr Lys Ser
1               5

<210>  12
<211>  5
<212>  PRT
<213>  Artificial

<220>
<223>  Artificial Sequence

<220>
<221>  MOD_RES
<222>  (1)..(1)
<223>  N-Palmitoyl-Tyr

<400>  12

Tyr Gly Gly Phe Met

1               5


<210>  13
<211>  5
<212>  PRT
<213>  Artificial

<220>
<223>  Artificial Sequence

<400>  13

Val Val Arg Pro Pro
1               5


<210>  14
<211>  5
<212>  PRT
<213>  Artificial

<220>
<223>  Artificial Sequence


<220>
<221>  MOD_RES
<222>  (1)..(1)
<223>  N-Palmitoyl-Tyr

<400>  14

Tyr Gly Gly Phe Leu
1               5


<210>  15
<211>  5
<212>  PRT
<213>  Artificial

<220>
<223>  Artificial Sequence


<220>
<221>  MOD_RES
<222>  (1)..(1)
<223>  N-Benzyloxycarbonyl-Gly

<400>  15

Gly Pro Phe Pro Leu
1               5

<210>  16
<211>  5
<212>  PRT
<213>  Artificial

<220>
<223>  Artificial Sequence

<400>  16

Gly Pro Phe Pro Leu
1               5


<210>  17
<211>  6
<212>  PRT
<213>  Artificial

<220>
<223>  Artificial Sequence

<400>  17

Val Gly Val Ala Pro Gly
1               5


<210>  18
<211>  6
<212>  PRT
<213>  Artificial

<220>
<223>  Artificial Sequence


<220>
<221>  MOD_RES
<222>  (1)..(1)
<223>  N-Palmitoyl-VAL

<400>  18

Val Gly Val Ala Pro Gly
1               5


<210>  19
<211>  6
<212>  PRT
<213>  Artificial

<220>
<223>  Peptide 02 Vincience

<400>  19

Ala Asp Leu Lys Pro Thr
1               5


<210>  20
<211>  6
<212>  PRT
<213>  Artificial

<220>
<223>  MOD_RES


<220>
<221>  MOD_RES
<222>  (4)..(4)
<223>  Xaa=Methylnorleucin

<220>
<221>  MOD_RES
<222>  (5)..(5)
<223>  Xaa=Homophenylalanin

<400>  20

Ala Arg His Xaa Xaa Trp
1               5


<210>  21
<211>  6
<212>  PRT
<213>  Artificial

<220>
<223>  Hexapeptide-1

<400>  21

Ala Arg His Leu Phe Trp
1               5


<210>  22
<211>  6
<212>  PRT
<213>  Artificial

<220>
<223>  Artificial Sequence

<400>  22

Val Val Arg Pro Pro Pro
1               5

SEQUENCE LISTING

<110>  Henkel AG & Co. KGaA

<120>  Antioxidative Zusammensetzungen

<130>  H 08154 EP

<150>  DE 102008061340.1
<151>  2008-12-11

<160>  22

<170>  PatentIn version 3.3

<210>  1
<211>  4
<212>  PRT
<213>  Artificial

<220>
<223>  Artificial

<400>  1

Gly Gln Pro Arg
1


<210>  2
<211>  4
<212>  PRT
<213>  Artificial

<220>
<223>  N-Palmitoyl

<400>  2

Gly Gln Pro Arg
1


<210>  3
<211>  4
<212>  PRT
<213>  Artificial

<220>
<223>  Artificial

<400>  3

Ala Gln Thr Arg
1

<210>  4
<211>  4
<212>  PRT
<213>  Artificial

<220>
<223>  tetrapeptide


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Cirullin, Canavanin.

<220>
<221>  misc_feature
<222>  (4)..(4)
<223>  Xaa chosen from Pro or Arg and amino acids that can replace Pro
       or Arg, Arg replacing amino acids are preferably Pro, Lys His,
       Desmosin or Isodesmosin.

<400>  4

Xaa Xaa Arg Xaa
1


<210>  5
<211>  4
<212>  PRT
<213>  Artificial

<220>
<223>  Artificial Sequence


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Cirullin, Canavanin.

<220>
<221>  misc_feature

<222>  (3)..(3)
<223>  Xaa chosen from Pro or Arg and amino acids that can replace Pro
       or Arg, Arg replacing amino acids are preferably Pro, Lys His,
       Desmosin or Isodesmosin.

<400>  5

Xaa Xaa Xaa Pro
1



<210>  6
<211>  4
<212>  PRT
<213>  Artificial

<220>
<223>  Artificial Sequence



<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Cirullin, Canavanin.

<220>
<221>  misc_feature
<222>  (4)..(4)
<223>  Xaa chosen from Pro or Arg and amino acids that can replace Pro
       or Arg, Arg replacing amino acids are preferably Pro, Lys His,
       Desmosin or Isodesmosin.

<400>  6

Xaa Xaa Pro Xaa
1



<210>  7
<211>  4
<212>  PRT
<213>  Artificial

<220>
<223>  Artificial Sequence



<220>
<221>  misc_feature
<222>  (1)..(1)

<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Cirullin, Canavanin.

<400>  7

Xaa Xaa Pro Arg
1


<210>  8
<211>  4
<212>  PRT
<213>  Artificial

<220>
<223>  Artificial Sequence


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Cirullin, Canavanin.

<220>
<221>  misc_feature
<222>  (3)..(3)
<223>  Xaa chosen from Pro or Arg and amino acids that can replace Pro
       or Arg, Arg replacing amino acids are preferably Pro, Lys His,
       Desmosin or Isodesmosin.

<400>  8

Xaa Xaa Xaa Arg
1


<210>  9
<211>  4
<212>  PRT
<213>  Artificial

<220>
<223>  Artificial Sequence

<400>  9

Val Val Arg Pro
1


<210>  10
<211>  5
<212>  PRT
<213>  Artificial

<220>
<223>  preferably with N-Acyl or ester-bond

<400>  10

Lys Thr Thr Lys Ser
1               5


<210>  11
<211>  5
<212>  PRT
<213>  Artificial

<220>
<223>  Artificial Sequence


<220>
<221>  MOD_RES
<222>  (1)..(1)
<223>  N-Palmitoyl-Lys

<400>  11

Lys Thr Thr Lys Ser
1               5


<210>  12
<211>  5
<212>  PRT
<213>  Artificial

<220>
<223>  Artificial Sequence


<220>
<221>  MOD_RES
<222>  (1)..(1)
<223>  N-Palmitoyl-Tyr

<400>  12

Tyr Gly Gly Phe Met

1               5


<210>  13
<211>  5
<212>  PRT
<213>  Artificial

<220>
<223>  Artificial Sequence

<400>  13

Val Val Arg Pro Pro
1               5


<210>  14
<211>  5
<212>  PRT
<213>  Artificial

<220>
<223>  Artificial Sequence


<220>
<221>  MOD_RES
<222>  (1)..(1)
<223>  N-Palmitoyl-Tyr

<400>  14

Tyr Gly Gly Phe Leu
1               5


<210>  15
<211>  5
<212>  PRT
<213>  Artificial

<220>
<223>  Artificial Sequence


<220>
<221>  MOD_RES
<222>  (1)..(1)
<223>  N-Benzyloxycarbonyl-Gly

<400>  15

Gly Pro Phe Pro Leu
1               5

<210>  16
<211>  5
<212>  PRT
<213>  Artificial

<220>
<223>  Artificial Sequence

<400>  16

Gly Pro Phe Pro Leu
1               5


<210>  17
<211>  6
<212>  PRT
<213>  Artificial

<220>
<223>  Artificial Sequence

<400>  17

Val Gly Val Ala Pro Gly
1               5


<210>  18
<211>  6
<212>  PRT
<213>  Artificial

<220>
<223>  Artificial Sequence


<220>
<221>  MOD_RES
<222>  (1)..(1)
<223>  N-Palmitoyl-VAL

<400>  18

Val Gly Val Ala Pro Gly
1               5


<210>  19
<211>  6
<212>  PRT
<213>  Artificial

<220>
<223>  Peptide 02 Vincience

<400>  19

Ala Asp Leu Lys Pro Thr
1               5


<210>  20
<211>  6
<212>  PRT
<213>  Artificial

<220>
<223>  MOD_RES


<220>
<221>  MOD_RES
<222>  (4)..(4)
<223>  Xaa=Methylnorleucin

<220>
<221>  MOD_RES
<222>  (5)..(5)
<223>  Xaa=Homophenylalanin

<400>  20

Ala Arg His Xaa Xaa Trp
1               5


<210>  21
<211>  6
<212>  PRT
<213>  Artificial

<220>
<223>  Hexapeptide-1

<400>  21

Ala Arg His Leu Phe Trp
1               5


<210>  22
<211>  6
<212>  PRT
<213>  Artificial

<220>
<223>  Artificial Sequence

<400>  22

Val Val Arg Pro Pro Pro
1               5